# EUROPÄISCHE PATENTSCHRIFT

(11) **EP 2 132 167 B1**
(45) Veröffentlichungstag und Bekanntmachung des Hinweises auf die Patenterteilung: **25.12.2013**
(21) Anmeldenummer: 08717291.2
(22) Anmeldetag: 29.02.2008
(51) Int. Cl.: C07C 253/30, C07D 487/04

(54) **VERFAHREN ZUR HERSTELLUNG VON beta-KETONITRILEN**
METHOD FOR THE PRODUCTION OF beta-KETONITRILES
PROCÉDÉ POUR LA PRODUCTION DE béta-CETONITRILES

(30) Priorität: 02.03.2007 EP 07103412
(43) Veröffentlichungstag der Anmeldung: 16.12.2009
(73) Patentinhaber: BASF SE, 67056 Ludwigshafen (DE)
(72) Erfinder: VOGELBACHER, Uwe Josef, 67071 Ludwigshafen (DE); OTTO, Peter, 67063 Ludwigshafen (DE); RACK, Michael, 69214 Eppelheim (DE)
(86) Internationale Anmeldenummer: PCT/EP2008/052514
(87) Internationale Veröffentlichungsnummer: WO 2008/107397

(56) Entgegenhaltungen:
- WO-A-2006/087325
- WO-A-2006/092412
- WO-A-2007/099317
- YAOHUI JI, WILLIAM C. TRENKLE, JAMES V. VOWLES: "A High-Yielding Preparation of beta-Ketonitriles" ORGANIC LETTERS, Bd. 8, Nr. 6, 23. Februar 2006 (2006-02-23), Seiten 1161-1163, XP002482394
- R.D. HAWORTH, C.R. MAVIN, G. SHELDRICK: "The Constituents of Guaiacum Resin. Part II. Synthesis of dl-Guaiaretic Acid Dimethyl Ether." JOURNAL OF THE CHEMICAL SOCIETY, 1934, Seiten 1423-1429, XP008092322
- FRASER F. FLEMING, VENUGOPAL GUDIPATI, OMAR W. STEWARD: "omega-Halonitriles: Domino Cyclizations to Oxa- and Carbocyclic Nitriles"" JOURNAL OF ORGANIC CHEMSITRY, Bd. 68, 16. April 2003 (2003-04-16), Seiten 3943-3946, XP002482395

## Beschreibung

Die vorliegende Erfindung betrifft ein Verfahren zur Herstellung von ß-Ketonitrilen der allgemeinen Formel I 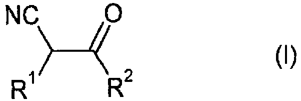 worin
- R¹: für C₂-C₁₂-Alkyl, C₂-C₁₂-Alkenyl, C₂-C₁₂-Alkinyl, C₃-C₈-Cycloalkyl, C₃-C₈-Cycloalkyl-C₁-C₄-alkyl, Phenyl-C₁-C₄-alkyl, Phenoxy-C₁-C₄-alkyl oder Benzyloxy-C₁-C₄-alkyl steht, wobei C₂-C₁₂-Alkyl durch einen der Reste R^{A} substituiert sein kann, die C₃-C₈-Cycloalkylgruppen in den vorgenannten Resten durch 1, 2, 3, 4 oder 5 Reste R^{B} substituiert sein können und die Phenylgruppen in den vorgenannten Resten durch 1, 2, 3, 4 oder 5 Reste R^{C} substituiert sein können;
R^{A} ausgewählt ist unter C₁-C₁₀-Alkoxy, C₁-C₆-Alkoxy-C₁-C₄-alkoxy, C₁-C₁₀-Alkylthio, NH₂, C₁-C₁₀-Alkylamino und Di-C₁-C₁₀-alkylamino;
R^{B} ausgewählt ist unter Fluor, C₁-C₄-alkyl, C₁-C₄-Perfluoralkyl und den für R^{A} angegebenen Bedeutungen;
R^{C} ausgewählt ist unter Fluor, Nitro, C₁-C₄-alkyl, C₁-C₄-Perfluoralkyl und den für R^{A} angegebenen Bedeutungen; wobei R¹ wenigstens 3 C-Atome aufweist;
- R²: für C₁-C₈-Alkyl steht,

ß-Ketonitrile, insbesondere solche der allgemeinen Formel I, sind interessante Ausgangsmaterialien für die Herstellung heterocyclischer Wirkstoffe, insbesondere für die Herstellung von fungizidwirksamen 7-Aminoazolopyrimidinen (siehe z. B. EP-A-141317, WO2006/087325).

Die Herstellung von ß-Ketonitrilen erfolgt üblicherweise durch Kondensation von Nitrilen, welche in der α-Position zur Nitrilgruppe Wasserstoffatome aufweisen, mit Carbonsäureestern in Gegenwart einer Base. Diese Reaktion ist für die Herstellung der ß-Ketonitrile der Formel I in dem folgenden Schema 1 dargestellt. In Schema 1 haben die Variablen R¹ und R² die zuvor genannten Bedeutungen. R steht für einen über ein Kohlenstoffatom gebundenen, inerten Rest mit in der Regel 1 bis 12 C-Atomen. Schema 1:

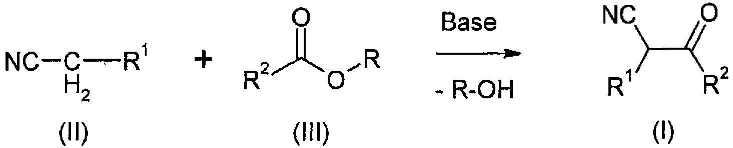

So beschreibt beispielsweise die EP 141317 die Herstellung von ß-acylierten Alkylcyaniden durch Umsetzung von Alkylcyaniden mit Carbonsäureestern und starken Basen, beispielsweise Alkalimetallhydriden, Alkalimetallamiden und Metallalkylen. Derartige Basen sind jedoch vergleichsweise teuer und schwierig zu handhaben, was eine Übertragung dieser Reaktion in den technischen Maßstab erschwert.

Die US2004/0171863 wiederum beschreibt die Herstellung von ß-Ketonitrilen durch Umsetzung eines Carbonsäureesters mit einem Alkylnitril, das in der α-Position zwei Wasserstoffatome trägt, und einer Base bei Temperaturen von 145 bis 300 °C in einem geschlossenen Gefäß, wobei man das Alkalimetallsalz des ß-Ketonitrils erhält, welches man anschließend mit einer Säure neutralisiert. Die dort angegebenen Reaktionsbedingungen führen jedoch nur zu mäßigen Ausbeuten, insbesondere bei Nitrilen mit vier oder mehr Kohlenstoffatomen.

Die WO2006/087325 wiederum beschreibt die Umsetzung von Decannitril mit Methoxyessigsäuremethylester und Kalium-tert.-butylat in wasserfreiem Dimethylformamid. Die gefundenen Ausbeuten sind nicht zufriedenstellend. Eigene Untersuchungen der Anmelderin haben zudem gezeigt, dass bei Anwendung höherer Temperaturen verstärkt Nebenprodukte gebildet werden, die sich nur schlecht abtrennen lassen.

Ein weiteres Verfahren zur Synthese von ß-Ketonitrilen wurde in Organic Letters 2006, 6, 1161-1163 beschrieben. Nach dem Verfahren lassen sich die Zielverbindungen in hohen Ausbeuten herstellen, wobei das Nitril zunächst bei Raumtemperatur in Gegenwart von Kalium-tert.-amylat deprotoniert wird und anschließend der Carbosäureester ebenfalls bei Raumtemperatur zugegeben wird. Das Reaktionsgemisch wird anschließend bei Raumtemperatur gerührt. Dieses Vorgehen hat den Nachteil, dass es den Einsatz von hohen Überschüssen des Carbonsäureesters erfordert.

Der vorliegenden Erfindung liegt daher die Aufgabe zu Grunde, ein Verfahren zur Herstellung von ß-Ketonitrilen der allgemeinen Formel I bereitzusteilen, das die Probleme des Standes der Technik löst. Insbesondere sollte es den Einsatz kostengünstiger Alkoholate erlauben und hierbei die gewünschten Produkte der Formel I mit hoher Ausbeute und guten Reinheiten liefern.

Diese Aufgabe wird durch ein Verfahren gelöst, welches die Umsetzung eines Nitrils der Formel II 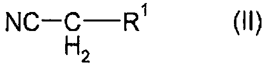 worin R¹ eine der zuvor genannte Bedeutungen aufweist, mit einem Carbonsäureester der Formel III umfass 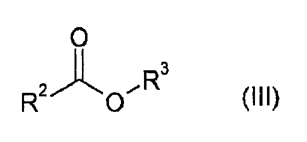 t,
worin R² eine der zuvor genannten Bedeutungen aufweist und R³ für C₁-C₁₂-Alkyl, C₁-C₄-Alkoxy-C₁-C₄-alkyl, C₃-C₈-Cycloalkyl, C₃-C₈-Cycloalkyl-C₁-C₄-alkyl, Phenyl-C₁-C₄-alkyl, Phenoxy-C₁-C₄-alkyl oder Benzyloxy-C₁-C₄-alkyl steht,
wobei man die Umsetzung in einem inerten Lösungsmittel durchführt, das unter aliphatischen Kohlenwasserstoffen, cycloaliphatischen Kohlenwasserstoffen, aromatischen Kohlenwasserstoffen, Dialkylethern, alicyclischen Ethern und deren Gemischen ausgewählt ist,
wobei man die Umsetzung in Gegenwart eines Kaliumalkoholats durchführt und wenigstens 80 % des Nitrils der Formel II der Umsetzung unter Reaktionsbedingungen zuführt, wobei die Zugabe des Nitrils der Formel II und des Esters der Formel III parallel stattfindet.

Gegenstand der Erfindung ist somit ein Verfahren zur Herstellung von ß-Ketonitrilen der allgemeinen Formel I, wie eingangs definiert, umfassend die Umsetzung eines Nitrils der allgemeinen Formel II mit einem Carbonsäureester der allgemeinen Formel III, das dadurch gekennzeichnet ist, dass man die Umsetzung in Gegenwart eines Kaliumalkoholats durchführt und wenigstens 80 % des Nitrils der Formel II der Umsetzung unter Reaktionsbedingungen zugeführt werden, wobei die Zugabe des Nitrils der Formel II und des Esters der Formel III parallel stattfindet.

Das erfindungsgemäße Verfahren liefert auch bei Einsatz des gut zu handhabenden Kaliumalkoholats ß-Ketonitrile in hohen Ausbeuten und mit guter Selektivität. Das erfindungsgemäße Verfahren eignet sich zur Herstellung von ß-Ketonitrilen der Formel I, worin R¹ drei oder mehr Kohlenstoffatome, insbesondere fünf oder mehr Kohlenstoffatome aufweist. Derartige ß-Ketonitrile sind durch die in Schema 1 gezeigte Reaktionssequenz auf Grund der Reaktionsträgheit der zu Grunde liegenden Nitrile der Formel II auf dem in Schema 1 gezeigten Wege nach den Methoden des Standes der Technik nur mit schlechten Ausbeuten erhältlich, insbesondere wenn man Alkoholate als Basen einsetzt.

Bei den in den voranstehenden und nachfolgenden Formeln angegebenen Definitionen der Substituenten R¹, R², R3, R⁴ und R⁵ sowie R^{A}, R^{B} und R^{C} wurden Sammelbegriffe verwendet, die allgemein repräsentativ für konkrete organische Reste stehen. Hierbei geben in dem Ausdruck Cₙ-Cₘ die Variablen n und m die jeweils mögliche Anzahl der Kohlenstoffatome in dem jeweiligen Rest an. Insbesondere bedeuten:

Alkyl, sowie die Alkylteile in Alkylthio, Alkylamino und Dialkylamino: ein linearer oder verzweigter Kohlenwasserstoffrest mit in der Regel ein bis zwölf Kohlenstoffatomen, oder 1 bis 4 Kohlenstoffatomen, 1 bis 6 Kohlenstoffatomen oder 5 bis 12 Kohlenstoffatomen, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere, 2-Ethylhexyl, 3,5,5-Trimethylhexyl, 3,5-Dimethylhexyl, n-Heptyl, 1-Methylheptyl, 2-Methylheptyl, 2-Ethylheptyl, 2-Propylheptyl, n-Octyl, 1-Methyloctyl, 2-Methyloctyl, n-Nonyl, 1-Methylnonyl, 2-Methylnonyl, n-Decyl, 1-Methyldecyl, n-Undecyl, 1-Methylundecyl und n-Dodecyl.

Alkenyl bedeutet dementsprechend einen linearen oder verzweigten, einfach ethylenisch ungesättigten Kohlenwasserstoffrest mit in der Regel zwei bis zwölf C-Atomen wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-, 2-, 3-, 4-, 5-oder 6-Heptenyl, 1-, 2-, 3-, 4-, 5-, 6- oder 7-Octenyl, 1-, 2-, 3-, 4-, 5-, 6-, 7- oder 8-Nonenyl, 1-, 2-, 3-, 4-, 5-, 6-, 7-, 8- oder 9-Decenyl.

Alkinyl steht für einen linearen oder verzweigten acetylenisch ungesättigten Kohlenwasserstoffrest mit in der Regel zwei bis zwölf C-Atomen wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 1-Ethyl-1-methyl-2-propinyl, 1-, 2-, 3-, 4-, 5- oder 6-Heptinyl, 1-, 2-, 3-, 4-, 5-, 6- oder 7-Octinyl, 1-, 2-, 3-, 4-, 5-, 6-, 7- oder 8-Noninyl, 1-, 2-, 3-, 4-, 5-, 6-, 7-, 8- oder 9-Decinyl.

Cycloalkyl steht für einen mono- oder bicyclischen Kohlenwasserstoffrest mit in der Regel drei bis acht C-Atomen wie Cyclopropyl, Cyclobutyl, Cyclopentyl, Cyclohexyl, Cycloheptyl, Cyclooctyl, Bicyclo[3.3.0]octyl, Bicyclo[2.2.1]heptyl und Bicyclo[3.2.1]octyl.

Cycloalkyl-C₁-C₄-alkyl steht für einen Alkylrest mit einem bis vier Kohlenstoffatomen, der einen Cycloalkylrest, wie oben definiert, mit vorzugsweise drei bis acht C-Atomen trägt.

Phenyl-C₁-C₄-alkyl steht für einen Alkylrest mit einem bis vier C-Atomen, der einen Phenylrest trägt, beispielsweise für Benzyl, 1-Phenylethyl und 2-Phenylethyl.

Phenoxy-C₁-C₄-alkyl steht für einen Alkylrest mit einem bis vier C-Atomen, der einen Phenoxy-Rest als Substituenten trägt. Dementsprechend bedeutet Benzyloxy-C₁-C₄-alkyl einen Alkylrest mit einem bis vier C-Atomen, der einen Benzyloxy-Rest trägt.

Alkoxy steht für einen über einen Sauerstoff gebundenen Alkylrest mit einem bis zehn und insbesondere einem bis acht Kohlenstoffatomen wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexyloxy, 1,1-Dimethylpropoxy, 1,2-Dimethylpropoxy, 1-Methylpentyloxy, 2-Methylpentyloxy, 3-Methylpentyloxy, 4-Methylpentyloxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy und deren Isomere, 2-Ethylhexyloxy, 3,5,5-Trimethylhexyloxy, 3,5-Dimethylhexyloxy, n-Heptyloxy, 1-Methylheptyloxy, 2-Methylheptyloxy, 2-Ethylheptyloxy, 2-Propylheptyloxy, n-Octyloxy, 1-Methyloctyloxy, 2-Methyloctyloxy, n-Nonyloxy, 1-Methylnonyloxy, 2-Methylnonyloxy und n-Decyloxy.

Alkylthio steht dementsprechend für einen linearen oder verzweigten Alkylrest mit in der Regel einem bis zehn C-Atomen, der über ein Schwefelatom gebunden ist.

Alkylamino steht für einen linearen oder verzweigten Alkylrest mit einem bis acht C-Atomen, der über eine NH-Gruppe gebunden ist. Dementsprechend steht Dialkyl-amino für eine Gruppe NR₂, worin R gleich oder verschieden sein kann und für lineares oder verzweigtes Alkyl mit in der Regel einem bis zehn C-Atomen steht.

Alkoxyalkyl steht für einen Alkylrest mit in der Regel einem bis vier C-Atomen, der einen Alkoxy-Rest, wie vorstehend definiert, trägt, z.B. für Methoxymethyl, Ethoxymethyl, -CH₂OCH₂-C₂H₅, -CH₂-OCH(CH₃)₂, n-Butoxymethyl, -CH₂-OCH(CH₃)-C₂H₅, -CH₂-OCH₂-CH(CH₃)₂, -CH₂-OC(CH₃), Methoxyethyl, Ethoxyethyl, -(CH₂)₂OCH₂-C₂H₅, -(CH₂)₂OCH(CH₃)₂, n-Butoxyethyl, -(CH₂)₂OCH(CH₃)-C₂H₅, -(CH₂)₂OCH₂-CH(CH₃)₂ oder -(CH₂)₂-OC(CH₃), 1-Methylbutoxymethyl, 2-Methylbutoxymethyl, 3-Methylbutoxymethyl, 2,2-Dimethylpropoxymethyl, 1-Ethylpropoxymethyl, Hexyloxymethyl, 1,1-Dimethylpropoxymethyl, 1,2-Dimethylpropoxymethyl, 1-Methylpentyloxymethyl, 2-Methylpentyloxymethyl, 3-Methylpentyloxymethyl, 4-Methylpentyloxymethyl, 1,1-Dimethylbutoxymethyl, 1,2-Dimethylbutoxymethyl, 1,3-Dimethylbutoxymethyl, 2,2-Dimethylbutoxymethyl, 2,3-Dimethylbutoxymethyl, 3,3-Dimethylbutoxymethyl, 1-Ethylbutoxymethyl, 2-Ethylbutoxymethyl, 1,1,2-Trimethylpropoxymethyl, 1,2,2-Trimethylpropoxymethyl, 1-Ethyl-1-methylpropoxymethyl, 1-Ethyl-2-methylpropoxymethyl und deren Isomere, 2-Ethylhexyloxymethyl, 3,5,5-Trimethylhexyloxymethyl, 3,5-Dimethylhexyloxymethyl, n-Heptyloxymethyl, 1-Methylheptyloxymethyl, 2-Methylheptyloxymethyl, 2-Ethylheptyloxymethyl, 2-Propylheptyloxymethyl, n-Octyloxymethyl, 1-Methyloctyloxymethyl, 2-Methyloctyloxymethyl, n-Nonyloxymethyl, 1-Methylnonyloxymethyl, 2-Methylnonyloxymethyl, n-Decyloxymethyl, 1-(1-Methylbutoxy)ethyl, 1-(2-Methylbutoxy)ethyl, 1-(3-Methylbutoxy)ethyl, 1-(2,2-Dimethylpropoxy)ethyl, 1-(1-Ethylpropoxy)ethyl, 1-(Hexyloxy)ethyl, 1-(1,1-Dimethylpropoxy)ethyl, 1-(1,2-Dimethylpropoxy)ethyl, 1-(1-Methylpentyloxy)ethyl, 1-(2-Methylpentyloxy)ethyl, 1-(3-Methylpentyloxy) ethyl, 1-(4-Methylpentyloxy)ethyl, 1-(1,1-Dimethylbutoxy)ethyl, 1-(1,2-Dimethylbutoxy)ethyl, 1-(,3-Dimethylbutoxy)ethyl, 1-(2,2-Dimethylbutoxy)ethyl, 1-(2,3-Dimethylbutoxy)ethyl, 1-(3,3-Dimethylbutoxy)ethyl, 1-(1-Ethylbutoxy)ethyl, 1-(2-Ethylbutoxy)ethyl, 1-(2-Ethylhexyloxy)ethyl, 1-(3,5,5-Trimethylhexyloxy)ethyl, 1-(3,5-Dimethylhexyloxy)ethyl, 1-(n-Heptyloxy)ethyl, 1-(1-Methylheptyloxy)ethyl, 1-(2-Propylheptyloxy)ethyl, 1-(n-Octyloxy)ethyl, 1-(1-Methyloctyloxy)ethyl, 1-(2-Methyloctyloxy)ethyl, 1-(n-Nonyloxy)ethyl, 1-(1-Methylnonyloxy)ethyl, 1-(2-Methylnonyloxy)ethyl, 1-(n-Decyloxy)ethyl, 2-(1-Methylbutoxy)ethyl, 2-(2-Methylbutoxy)ethyl, 2-(3-Methylbutoxy)ethyl, 2-(2,2-Dimethylpropoxy)ethyl, 2-(1-Ethylpropoxy)ethyl, 2-(Hexyloxy)ethyl, 2-(1,1-Dimethylpropoxy)ethyl, 2-(1,2-Dimethylpropoxy)ethyl, 2-(1-Methylpentyloxy)ethyl, 2-(2-Methylpentyloxy)ethyl, 2-(3-Methylpentyloxy) ethyl, 2-(4-Methylpentyloxy)ethyl, 2-(1,1-Dimethylbutoxy)ethyl, 2-1,2-Dimethylbutoxy)ethyl, 2-(,3-Dimethylbutoxy)ethyl, 2-(2,2-Dimethylbutoxy)ethyl, 2-(2,3-Dimethylbutoxy)ethyl, 2-(3,3-Dimethylbutoxy)ethyl, 2-(1-Ethylbutoxy)ethyl, 2-(2-Ethylbutoxy)ethyl, 2-(2-Ethylhexyloxy)ethyl, 2-(3,5,5-Trimethylhexyloxy)ethyl, 2-(3,5-Dimethylhexyloxy)ethyl, 2-(n-Heptyloxy)ethyl, 2-(1-Methylheptyloxy)ethyl, 2-(2-Propylheptyloxy)ethyl, 2-(n-Octyloxy)ethyl, 2-(1-Methyloctyloxy)ethyl, 2-(2-Methyloctyloxy)ethyl, 2-(n-Nonyloxy)ethyl, 2-(1-Methylnonyloxy)ethyl, 2-(2-Methylnonyloxy)ethyl und 2-(n-Decyloxy)ethyl.

Alkoxyalkoxy steht für einen Alkoxy-Rest mit in der Regel einem bis vier C-Atomen, der einen Alkoxy-Rest, wie vorstehend definiert, trägt, z. B. für Methoxymethoxy, Ethoxymethoxy, -OCH₂OCH₂-C₂H₅. -OCH₂-OCH(CH₃)₂, n-Butoxymethoxy, -OCH₂-OCH(CH₃)-C₂H₅, -OCH₂-OCH₂-CH(CH₃)₂, -OCH₂-OC(CH₃), Methoxyethoxy, Ethoxyethoxy, -O(CH₂)₂OCH₂-C₂H₅, -O(CH₂)₂OCH(CH₃)₂, n-Butoxyethoxy, -O(CH₂)₂OCH(CH₃)-C₂H₅, -O(CH₂)₂OCH₂-CH(CH₃)₂ oder -O(CH₂)₂-OC(CH₃) etc.

Perfluoralkyl steht für einen Alkylrest mit in der Regel einem bis vier C-Atomen, in dem die Wasserstoffatome durch Fluoratome ersetzt sind. Beispiele für Perfluoralkyl sind insbesondere Trifluormethyl, Pentafluorethyl, Heptafluorpropyl etc.

Erfindungsgemäß führt man bei der Umsetzung des Nitrils der Formel II mit dem Carbonsäureester der Formel III wenigstens 80 %, insbesondere wenigstens 90 %, besonders bevorzugt wenigstens 95 % und speziell die Gesamtmenge oder wenigstens 99 % des Nitrils der Formel II der Umsetzung unter Reaktionsbedingungen zu.

Der Begriff "unter Reaktionsbedingungen" ist dem Fachmann geläufig und bedeutet, dass in dem Reaktionsgefäß bzw. in der Reaktionszone, in welchem bzw. in welcher die gewünschte Umsetzung durchgeführt wird, Bedingungen herrschen, so dass die gewünschte Reaktion mit hinreichender Reaktionsgeschwindigkeit abläuft. Im Erfindungsgemäßen Verfahren bedeutet dies, dass in dem Reaktionsgefäß bzw. in der Reaktionszone, in dem (bzw. der) die Umsetzung des Nitrils II mit dem Ester III durchgeführt wird, Temperaturen herrschen, bei dem in Gegenwart des Kaliumalkoholats eine Reaktion des Nitrils II mit dem Carbonsäureester III unter Bildung des Kaliumsalzes der Verbindung I mit einer für die Umsetzung hinreichenden Geschwindigkeit ablaufen kann.

Die für die Umsetzung erforderlichen Temperaturen kann der Fachmann durch Routineexperimente ermitteln und liegen üblicherweise bei wenigstens 50 °C, insbesondere wenigstens 80 °C und besonders bevorzugt bei wenigstens 100 °C. Die Reaktionstemperaturen werden in der Regel 200 °C nicht überschreiten und liegen vorzugsweise im Bereich von 80 bis 180 °C und speziell im Bereich von 110 bis 150 °C.

Der Reaktionsdruck ist für die Umsetzung von untergeordneter Bedeutung. In der Regel erfolgt die Umsetzung in Reaktoren mit Druckausgleich zum Umgebungsdruck, so dass die Umsetzung bei Umgebungsdruck erfolgt. Aus technischen Gründen kann auch eine Umsetzung bei leicht verringertem Druck, z. B. im Bereich von 0,9 bar (absolut) bis Umgebungsdruck, oder bei leicht erhöhtem Druck, z. B. im Bereich von Umgebungsdruck bis 3,0 bar (absolut) von Vorteil sein.

Die Zugabe der gewünschten Menge an Nitril kann in einer oder mehren Portionen oder kontinuierlich über einen längeren Zeitraum mit konstanter oder sich ändernder Zugaberate erfolgen. Häufig wird man so vorgehen, dass man diejenige Menge an Nitril, die im Verlaufe der Reaktion zugegeben wird, über einen gewissen Zeitraum zugibt, der in der Regel im Bereich von fünf Minuten bis einer Stunde und insbesondere im Bereich von zehn Minuten bis dreißig Minuten liegt.

Für das erfindungsgemäße Verfahren hat es sich weiterhin als vorteilhaft erwiesen, wenn man die Hauptmenge, insbesondere wenigstens 80 % des Carbonsäureesters der Formel III der Umsetzung unter Reaktionsbedingungen zuführt. Gemäß einer ersten bevorzugten Ausführungsform der Erfindung gibt man wenigstens 90 % und insbesondere wenigstens 95 % und besonders bevorzugt die Gesamtmenge oder wenigstens 99 % des Carbonsäureesters der Formel III unter Reaktionsbedingungen zu. Gemäß einer ebenfalls bevorzugten Ausführungsform legt man 1 bis 20 Gew.-% und insbesondere 5 bis 15 Gew.-% des Carbonsäureesters im Reaktionsgefäß vor und gibt unter Reaktionsbedingungen die Restmenge an Carbonsäureester zu.

Sofern die Hauptmenge des Carbonsäureesters der Formel III im Verlaufe der Reaktion zugegeben wird, kann die Zugabe ebenfalls in einer Portion, in mehreren Portionen oder über einen längeren Zeitraum kontinuierlich mit konstanter oder sich ändernder Zugaberate erfolgen. Vorzugsweise erfolgt die Zugabe der gewünschten Menge an Carbonsäureester III über einen gewissen Zeitraum, der in der Regel fünf Minuten bis eine Stunde und insbesondere zehn Minuten bis dreißig Minuten beträgt.

Vorzugsweise erfolgt die Umsetzung des Nitrils II mit dem Carbonsäureester III in einem inerten Lösungsmittel. Beispiele für geeignete inerte Lösungsmittel sind aliphatische und cycloaliphatische Kohlenwasserstoffe wie Hexan, Heptan, Octan, Cyclohexan, Cycloheptan und Cyclooctan, aromatische Kohlenwasserstoffe, insbesondere Alkylaromaten wie Toluol, Xylole, 1,2-, 1,3- und 1,4-Dimethylbenzol und deren Gemische, Mesitylen, Ethylbenzol, Isopropylbenzol (Cumol), 1,2-, 1,3- und 1,4-Methylisopropylbenzol (Cymole) und deren Gemische, 1,3- und 1,4-Diisopropylbenzol und deren Gemische, 1,2-, 1,3- und 1,4-Diethylbenzol und deren Gemische, weiterhin Dialkylether und alicyclische Ether wie Di-n-propylether, Diisopropylether, Methyl-tert.-butylether, Ethyl-tert.-butylether, Ethylenglykoldimethylether, Ethylenglykoldiethylether, Diethylenglykoldimethylether, Triethylenglykoldimethylether, Tetrahydrofuran, 2-Methyltetrahydrofuran, Dioxan und Tetrahydropyran. Geeignet sind auch Gemische der vorgenannten inerten Lösungsmittel.

Bevorzugte inerte Lösungsmittel sind die vorgenannten Alkylaromaten und deren Gemische mit davon verschiedenen inerten Lösungsmitteln, beispielsweise mit aliphatischen und alicyclischen Kohlenwasserstoffen oder den vorgenannten Ethern. Vorzugsweise umfasst das inerte Lösungsmittel zu wenigstens 80 Gew.-% und insbesondere wenigstens 90 Gew.-%, bezogen auf die Gesamtmenge an inertem Lösungsmittel, einen oder mehrere Alkylaromaten oder ein Gemisch aus wenigstens einem Alkyl-aromaten mit einem oder mehreren aliphatischen oder cycloaliphatischen Kohlenwasserstoffen. Ganz besonders bevorzugt umfasst das inerte Lösungsmittel zu wenigstens 80 Gew.-% und insbesondere zu wenigstens 90 Gew.-% Alkylaromaten, insbesondere Xylol oder Mesitylen. Vorzugsweise weist das Lösungsmittel oder Lösungsmittelgemisch einen Siedepunkt in dem oben genannten Temperaturbereich auf.

Die Menge an Lösungsmittel wird in der Regel so gewählt, das die Gesamtmenge an Einsatzstoffen (d. h. die Gesamtkonzentration an Verbindungen II, III und Kaliumalkoholat) im Bereich von 20 bis 80 Gew.-%, insbesondere im Bereich von 30 bis 70 Gew.-%, bezogen auf die Gesamtmenge an Einsatzstoffen und Lösungsmittel liegt.

Erfindungsgemäß erfolgt die Umsetzung in Gegenwart eines Kaliumalkoholats. Das Kaliumalkoholat dient dabei als Base. Gegebenenfalls können auch geringe Mengen anderer Basen bei der Umsetzung zugegen sein. In der Regel macht das Kaliumalkoholat wenigstens 70 Gew.-%, vorzugsweise wenigstens 80 Gew.-% und insbesondere wenigstens 90 Gew.-% der eingesetzten Base aus. Geeignete Kallumalkoholate sind insbesondere die Kaliumsalze von C₁-C₆-Alkanolen wie Kaliummethanolat, Kalium-ethanolat, Kaliumpropanolat, Kalium-n-butanolat, Kalium-tert.-butanolat, Kalium-2-butanolat und Kalium-(2-methyl-2-butanolat). Die Kaliumalkoholate können auch in Form von Gemischen eingesetzt werden. Geeignete weitere Basen sind insbesondere Natrium- und Kaliumsalze schwacher Säuren, wie Natrium- und Kaliumhydroxid, Natrium- und Kaliumcarbonat, Natriumalkoholate, insbesondere die Natriumsalze von C₁-C₆-Alkanolen und dergleichen, aber auch Natriumhydrid und Kaliumhydrid. Vorzugsweise macht das Kaliumalkoholat wenigstens 95 %, insbesondere wenigstens 98 %, bezogen auf die Gesamtmenge an eingesetzter Base aus. Geringe Mengen an Kaliumhydroxid, die aus der Hydrolyse des Kaliumalkoholats resultieren, stören im Allgemeinen nicht. Vorzugsweise beträgt ihr Anteil jedoch nicht mehr als 3 Gew.-% und insbesondere nicht mehr als 1,5 Gew.-%, bezogen auf die Gesamtmenge an Base. Die Menge an eingesetztem Kaliumalkoholat beträgt vorzugsweise wenigstens 0,9 Mol, insbesondere wenigstens 1 Mol und besonders bevorzugt wenigstens 1,1 Mol pro Mol Nitril der Formel II. Da sich größere Mengen an Base in der Regel nicht nachteilig für die Reaktion auswirken, kann die Menge an Kaliumalkoholat auch 2,5, 3 oder 4 Mol oder mehr, bezogen auf ein Mol des Nitrils II betragen. Vorzugsweise liegt das Molverhältnis von Kaliumalkoholat zu der Gesamtmenge an Nitril der Formel II im Bereich von 1,1 : 1 bis 4 : 1, insbesondere 2: 1 bis 4 : 1.

Für die Reaktion hat es sich weiterhin als vorteilhaft erwiesen, wenn das zur Reaktion eingesetzte organische Lösungsmittel geringe Mengen eines C₁-C₄-Alkanols enthält. Die Menge an C₁-C₄-Alkanol beträgt in der Regel nicht mehr als 50 Mol-% und insbesondere nicht mehr als 20 Mol-% und liegt beispielsweise im Bereich von 1 bis 50 Mol-% oder 1 bis 20 Mol-%, bezogen auf die Gesamtmenge des in der Reaktion eingesetzten Esters der Formel III. Dabei hat es sich als vorteilhaft erwiesen, wenn man diese Menge an Alkanol im Reaktionsgefäß vorlegt.

Das Mol-Verhältnis von Nitril der Formel II zu Ester der Formel III liegt typischerweise im Bereich von 1 : 3 bis 1,5 : 1, vorzugsweise im Bereich von 1 : 2 bis 1 : 1 und insbesondere im Bereich von 1 : 1,1 bis 1 : 1,5.

Die Durchführung der Reaktion erfolgt in den hierfür üblichen Reaktionsgefäßen, die in der Regel mit üblichen Mitteln zur durch Mischung der Reaktanden, beispielsweise Rührern, Mitteln für die Zufuhr der Reaktanden der Formeln II und III, Mitteln zur Kontrolle der Reaktionstemperatur und des Reaktionsdrucks und dergleichen ausgerüstet sind. Die Reaktion kann kontinuierlich oder diskontinuierlich, d. h. absatzweise durchgeführt werden, wobei Letzteres bevorzugt ist.

Bei der üblichen absatzweisen Reaktionsführung wird man üblicherweise in einem Reaktionskessel arbeiten, der mit Mitteln für die Zufuhr des Nitrils der Formel II und des Esters der Formel III und geeigneten Mitteln für die Durchmischung der Reaktanden, beispielsweise Rührern, ausgestattet ist. Die Reaktion kann bei Umgebungsdruck oder bei erhöhtem Druck durchgeführt werden. In der Regel wird man bei der absatzweisen Durchführung der Reaktion so vorgehen, dass man eine Teilmenge oder die Gesamtmenge des Kaliumalkoholats, vorzugsweise wenigstens 80 %, bezogen auf die Gesamtmenge an Kaliumalkoholat, gegebenenfalls mit einer Teilmenge oder der Gesamtmenge an inertem Lösungsmittel und gegebenenfalls eine Teilmenge des Esters der Formel III mit bis zu 20 % des Nitrils der Formel II und gegebenenfalls das C₁-C₄-Alkanol im Reaktionsgefäß vorlegt. Anschließend erwärmt man die so erhaltene Mischung auf Reaktionstemperatur und beginnt dann mit der Zugabe der Restmenge an Kaliumalkoholat und der Restmenge an inertem Lösungsmittel. Vorzugsweise werden das Alkoholat und das inerte Lösungsmittel, sofern erwünscht, vollständig im Reaktor vorgelegt. Die Teilmenge oder insbesondere die Hauptmenge des Esters der Formel III wird unter Reaktionsbedingungen zugegeben und erfolgt parallel zu der Zugabe des Nitrils II, d. h. der Beginn und das Ende der Zugabe des Esters III weicht nicht oder nur wenige Minuten von Beginn bzw. Ende der Zugabe des Nitrils der Formel II ab (häufig nicht mehr als 5 min. insbesondere nicht mehr als 2 min). Insbesondere beginnt man zeitgleich (< ± 2 min.) mit der Zugabe des Nitrils II und des Esters III und beendet die Zugabe ebenfalls zeitgleich (< ± 2 min.). In der Regel schließt sich an die Beendigung der Zugabe des Nitrils II und des Esters III noch eine Nachreaktionsphase an, bei der das Reaktionsgemisch für einen gewissen Zeitraum bei der Reaktionstemperatur gehalten wird. Dieser Zeitraum beträgt in der Regel wenigstens dreißig Minuten und wird in der Regel 24 Stunden nicht überschreiten. Insbesondere liegt er im Bereich von 1 bis 12 Stunden und speziell im Bereich von 2 bis 8 Stunden.

Nach Beendigung der Reaktion kann man das Reaktionsgemisch in üblicher Weise aufarbeiten und, sofern erforderlich, das ß-Ketonitril der Formel I isolieren.

Zur Aufarbeitung des Reaktionsgemischs wird man in der Regel zunächst das gebildete Kaliumsalz des ß-Ketonitrils der allgemeinen Formel I und gegebenenfalls vorhandenes Alkoholat neutralisieren. Hierzu vermischt man das Reaktionsgemisch mit Wasser oder einer wässrigen Säure, beispielsweise mit einer wässrigen Salzsäure oder einer wässrigen Schwefelsäure. Vorzugsweise erfolgt das Vermischen unter pH-Kontrolle, wobei der pH-Wert einen Wert von vorzugsweise pH = 2 und insbesondere einen Wert von pH = 3 nicht unterschreiten sollte. Hierzu kann man beispielsweise so vorgehen, dass man eine verdünnte wässrige Säure in das Reaktionsgemisch in einer Menge einbringt, bis der pH-Wert der erhaltenen wässrigen Phase im Bereich von pH 2 bis 9 und insbesondere im Bereich pH 3 bis 8 liegt. Alternativ kann man so vorgehen, dass man das Reaktionsgemisch in Wasser oder in eine wässrige Säure einbringt und gegebenenfalls durch Zugabe von Säure den pH-Wert der wässrigen Phase auf einen pH-Wert im Bereich von pH 2 bis 9 und insbesondere pH 3 bis 8 nachreguliert. Die organische Phase enthält dann das gewünschte ß-Ketonitril der Formel I, gegebenenfalls gelöst in einem organischen Lösungsmittel.

Das ß-Ketonitril der Formel I kann aus der organischen Phase, gegebenenfalls nach Trocknen, in üblicher Weise isoliert werden, beispielsweise durch Abdestillieren des organischen Lösungsmittels. Das zurückbleibende ß-Ketonitril kann dann einer weiteren Aufreinigung unterworfen werden. Es hat sich jedoch gezeigt, dass unter den erfindungsgemäßen Reaktionsbedingungen das ß-Ketonitril der Formel I in einer Reinheit anfällt, die für weitere Umsetzungen in der Regel ausreichend ist. Häufig kann sogar auf eine Entfernung des organischen Lösungsmittels verzichtet werden.

Das erfindungsgemäße Verfahren eignet sich insbesondere zur Herstellung von ß-Ketonitrilen der allgemeinen Formel I, worin R¹ für einen Substituenten steht, der wenigstens vier C-Atome trägt. Insbesondere steht R¹ in den Formeln I und II für C₄-C₁₂-Alkyl, C₃-C₁₂-Alkenyl, Benzyl, C₅-C₈-Cycloalkyl oder C₁-C₁₀-Alkoxy-C₁-C₆-alkyl steht. Besonders bevorzugt steht R¹ für C₆-C₁₂-Alkyl. Ebenfalls bevorzugt steht R¹ für C₃-C₁₂-Alkenyl.

Das erfindungsgemäße Verfahren eignet sich zur Herstellung von ß-Ketonitrilen der allgemeinen Formel I, worin R² für C₁-C₈-Alkyl und speziell C₁-C₄-Alkyl steht.

Beispiele für geeignete ß-Ketonitrile der Formel I sind die in der Tabelle 1 angegebenen Verbindungen der allgemeinen Formel I, worin R¹ und R² jeweils die in einer Zeile der Tabelle angegebenen Bedeutungen aufweisen:

**Tabelle 1:**

| | R¹ | R² |
|---|---|---|
| I-1 | CH₂-C₆H₅ | CH₃ |
| I-2 | CH₂CH₂CH₃ | CH₃ |
| I-3 | CH₂CH₂CH₂CH₃ | CH₃ |
| I-4 | CH₂CH₂CH₂CH₂CH₃ | CH₃ |
| I-5 | CH₂CH₂CH₂CH₂CH₂CH₃ | CH₃ |
| I-6 | CH₂CH(CH₃)CH₂CH₂CH₃ | CH₃ |
| I-7 | CH₂CH(CH₂CH₃)₂ | CH₃ |
| I-8 | CH₂CH₂CH₂CH₂CH₂CH₂CH₃ | CH₃ |
| I-9 | CH₂CH₂CH₂CH₂CH₂CH₂CH₂CH₃ | CH₃ |
| I-10 | CH₂CH₂CH₂CH₂CH₂CH₂CH₂CH₂CH₃ | CH₃ |
| I-11 | CH₂CH₂CH₂CH₂CH₂CH₂CH₂CH₂CH₂CH₃ | CH₃ |
| I-12 | CH₂CH₂CH(CH₃)CH₂C(CH₃)₃ | CH₃ |
| I-13 | CH₂CH₂C(CH₃)₂CH₂C(CH₃)₃ | CH₃ |
| I-14 | (CH₂)₃-O-CH₃ | CH₃ |
| I-15 | (CH₂)₃-O-CH₂CH₃ | CH₃ |
| I-16 | (CH₂)₃-O-CH₂CH₂CH₃ | CH₃ |
| I-17 | (CH₂)₃-O-CH₂CH₂CH₂CH₃ | CH₃ |
| I-18 | (CH₂)₃-O-CH₂CH₂CH₂CH₂CH₃ | CH₃ |
| I-19 | (CH₂)₃-O-CH₂CH₂CH₂CH₂CH₂CH₃ | CH₃ |
| I-20 | (CH₂)₃-O-CH₂CH₂CH₂CH₂CH₂CH₂CH₃ | CH₃ |
| I-21 | (CH₂)₃-O-CH₂CH₂CH₂CH₂CH₂CH₂CH₂CH₃ | CH₃ |
| I-22 | (CH₂)₃-O-(CH₂)₈CH₃ | CH₃ |
| I-23 | (CH₂)₃-O-CH(CH₃)₂ | CH₃ |
| I-24 | (CH₂)₃-O-C(CH₃)₃ | CH₃ |
| I-25 | (CH₂)₃-O-CH₂C(CH₃)₃ | CH₃ |
| I-26 | (CH₂)₃-O-CH(CH₃)CH₂C(CH₃)₃ | CH₃ |
| I-27 | (CH₂)₃-O-CH(CH₂CH₃)CH₂C(CH₃)₃ | CH₃ |
| I-28 | (CH₂)₃-O-CH₂CH(CH₃)CH₂CH(CH₃)₂ | CH₃ |
| I-29 | (CH₂)₃-O-CH₂CH(CH₂CH₃)CH₂CH₂CH₃ | CH₃ |
| I-30 | (CH₂)₃-O-CH₂CH₂CH(CH₃)CH₂CH(CH₃)₂ | CH₃ |
| I-31 | (CH₂)₃-O-CH₂CH₂CH(CH₃)CH₂C(CH₃)₃ | CH₃ |
| I-32 | (CH₂)₃-O-CH₂CH₂CH(CH₃)CH₂CH₂CH(CH₃)₂ | CH₃ |
| I-33 | (CH₂)₃-O-CH₂CH₂CH(CH₃)(CH₂)₃CH(CH₃)₂ | CH₃ |
| I-34 | cyclo-C₅H₉ | CH₃ |
| I-35 | cyclo-C₆H₁₁ | CH₃ |
| I-36 | CH₂-C₆H₅ | CF₃* |
| I-37 | CH₂CH₂CH₃ | CF₃* |
| I-38 | CH₂CH₂CH₂CH₃ | CF₃* |
| I-39 | CH₂CH₂CH₂CH₂CH₃ | CF₃* |
| I-40 | CH₂CH₂CH₂CH₂CH₂CH₃ | CF₃* |
| I-41 | CH₂CH(CH₃)CH₂CH₂CH₃ | CF₃* |
| I-42 | CH₂CH(CH₂CH₃)₂ | CF₃* |
| I-43 | CH₂CH₂CH₂CH₂CH₂CH₂CH₃ | CF₃* |
| I-44 | CH₂CH₂CH₂CH₂CH₂CH₂CH₂CH₃ | CF₃* |
| I-45 | CH₂CH₂CH₂CH₂CH₂CH₂CH₂CH₂CH₃ | CF₃* |
| I-46 | CH₂CH₂CH₂CH₂CH₂CH₂CH₂CH₂CH₂CH₃ | CF₃* |
| I-47 | CH₂CH₂CH(CH₃)CH₂C(CH₃)₃ | CF₃* |
| I-48 | CH₂CH₂C(CH₃)₂CH₂C(CH₃)₃ | CF₃* |
| I-49 | cyclo-C₅H₉ | CF₃* |
| I-50 | cyclo-C₆H₁₁ | CF₃* |
| I-51 | CH₂CH₂CH₂CH₃ | CH₂CH₃ |
| I-52 | CH₂CH₂CH₂CH₂CH₃ | CH₂CH₃ |
| I-53 | CH₂CH₂CH₂CH₂CH₂CH₃ | CH₂CH₃ |
| I-54 | CH₂CH(CH₃)CH₂CH₂CH₃ | CH₂CH₃ |
| I-55 | CH₂CH(CH₂CH₃)₂ | CH₂CH₃ |
| I-56 | CH₂CH₂CH₂CH₂CH₂CH₂CH₃ | CH₂CH₃ |
| I-57 | CH₂CH₂CH₂CH₂CH₂CH₂CH₂CH₃ | CH₂CH₃ |
| I-58 | CH₂CH₂CH₂CH₂CH₂CH₂CH₂CH₂CH₃ | CH₂CH₃ |
| I-59 | CH₂CH₂CH₂CH₂CH₂CH₂CH₂CH₂CH₂CH₃ | CH₂CH₃ |
| I-60 | CH₂CH₂CH(CH₃)CH₂C(CH₃)₃ | CH₂CH₃ |
| I-61 | CH₂CH₂C(CH₃)₂CH₂C(CH₃)₃ | CH₂CH₃ |
| I-62 | (CH₂)₃-O-CH₃ | CH₂CH₃ |
| I-63 | (CH₂)₃-O-CH₂CH₃ | CH₂CH₃ |
| I-64 | (CH₂)₃-O-CH₂CH₂CH₃ | CH₂CH₃ |
| I-65 | (CH₂)₃-O-CH₂CH₂CH₂CH₃ | CH₂CH₃ |
| I-66 | (CH₂)₃-O-CH₂CH₂CH₂CH₂CH₃ | CH₂CH₃ |
| I-67 | (CH₂)₃-O-CH₂CH₂CH₂CH₂CH₂CH₃ | CH₂CH₃ |
| I-68 | (CH₂)₃-O-CH₂CH₂CH₂CH₂CH₂CH₂CH₃ | CH₂CH₃ |
| I-69 | (CH₂)₃-O-CH₂CH₂CH₂CH₂CH₂CH₂CH₂CH₃ | CH₂CH₃ |
| I-70 | (CH₂)₃-O-(CH₂)₈CH₃ | CH₂CH₃ |
| I-71 | (CH₂)₃-O-CH(CH₃)₂ | CH₂CH₃ |
| I-72 | (CH₂)₃-O-C(CH₃)₃ | CH₂CH₃ |
| I-73 | (CH₂)₃-O-CH₂C(CH₃)₃ | CH₂CH₃ |
| I-74 | (CH₂)₃-O-CH(CH₃)CH₂C(CH₃)₃ | CH₂CH₃ |
| I-75 | (CH₂)₃-O-CH(CH₂CH₃)CH₂C(CH₃)₃ | CH₂CH₃ |
| I-76 | (CH₂)₃-O-CH₂CH(CH₃)CH₂CH(CH₃)₂ | CH₂CH₃ |
| I-77 | (CH₂)₃-O-CH₂CH(CH₂CH₃)CH₂CH₂CH₃ | CH₂CH₃ |
| I-78 | (CH₂)₃-O-CH₂CH₂CH(CH₃)CH₂CH(CH₃)₂ | CH₂CH₃ |
| I-79 | (CH₂)₃-O-CH₂CH₂CH(CH₃)CH₂C(CH₃)₃ | CH₂CH₃ |
| I-80 | (CH₂)₃-O-CH₂CH₂CH(CH₃)CH₂CH₂CH(CH₃)₂ | CH₂CH₃ |
| I-81 | (CH₂)₃-O-CH₂CH₂CH(CH₃)(CH₂)₃CH(CH₃)₂ | CH₂CH₃ |
| I-82 | CH₂CH₂CH₂CH₃ | CH₂CH₂CH₃ |
| I-83 | CH₂CH₂CH₂CH₂CH₃ | CH₂CH₂CH₃ |
| I-84 | CH₂CH₂CH₂CH₂CH₂CH₃ | CH₂CH₂CH₃ |
| I-85 | CH₂CH(CH₃)CH₂CH₂CH₃ | CH₂CH₂CH₃ |
| I-86 | CH₂CH(CH₂CH₃)₂ | CH₂CH₂CH₃ |
| I-87 | CH₂CH₂CH₂CH₂CH₂CH₂CH₃ | CH₂CH₂CH₃ |
| I-88 | CH₂CH₂CH₂CH₂CH₂CH₂CH₂CH₃ | CH₂CH₂CH₃ |
| I-89 | CH₂CH₂CH₂CH₂CH₂CH₂CH₂CH₂CH₃ | CH₂CH₂CH₃ |
| I-90 | CH₂CH₂CH₂CH₂CH₂CH₂CH₂CH₂CH₂CH₃ | CH₂CH₂CH₃ |
| I-91 | CH₂CH₂CH(CH₃)CH₂C(CH₃)₃ | CH₂CH₂CH₃ |
| I-92 | CH₂CH₂C(CH₃)₂CH₂C(CH₃)₃ | CH₂CH₂CH₃ |
| I-93 | CH₂-O-CH₂CH₂CH₂CH₃* | CH₂CH₂CH₃ |
| I-94 | CH₂-O-CH₂CH₂CH₂CH₂CH₃* | CH₂CH₂CH₃ |
| I-95 | CH₂-O-CH₂CH₂CH₂CH₂CH₂CH₃* | CH₂CH₂CH₃ |
| I-96 | CH₂-O-CH₂CH₂CH₂CH₂CH₂CH₂CH₃* | CH₂CH₂CH₃ |
| I-97 | CH₂-O-CH₂CH₂CH₂CH₂CH₂CH₂CH₂CH₃* | CH₂CH₂CH₃ |
| I-98 | CH₂-O-CH₂CH₂CH₂CH₂CH₂CH₂CH₂CH₂CH₃* | CH₂CH₂CH₃ |
| I-99 | CH₂-O-C(CH₃)₃* | CH₂CH₂CH₃ |
| I-100 | CH₂-O-CH₂C(CH₃)₃* | CH₂CH₂CH₃ |
| I-101 | CH₂-O-CH(CH₃)CH₂C(CH₃)₃* | CH₂CH₂CH₃ |
| I-102 | CH₂-O-CH(CH₂CH₃)CH₂C(CH₃)₃* | CH₂CH₂CH₃ |
| I-103 | CH₂-O-CH₂CH(CH₃)CH₂CH(CH₃)₂* | CH₂CH₂CH₃ |
| I-104 | CH₂-O-CH₂CH(CH₂CH₃)CH₂CH₂CH₃* | CH₂CH₂CH₃ |
| I-105 | CH₂-O-CH₂CH₂CH(CH₃)CH₂CH(CH₃)₂* | CH₂CH₂CH₃ |
| I-106 | CH₂-O-CH₂CH₂CH(CH₃)CH₂C(CH₃)₃* | CH₂CH₂CH₃ |
| I-107 | CH₂-O-CH₂CH₂CH(CH₃)CH₂CH₂CH(CH₃)₂* | CH₂CH₂CH₃ |
| I-108 | CH₂-O-CH₂CH₂CH(CH₃)(CH₂)₃CH(CH₃)₂* | CH₂CH₂CH₃ |
| I-109 | (CH₂)₂-O-CH₂CH₂CH₃ | CH₂CH₂CH₃ |
| I-110 | (CH₂)₂-O-CH₂CH₂CH₂CH₃ | CH₂CH₂CH₃ |
| I-111 | (CH₂)₂-O-CH₂CH₂CH₂CH₂CH₃ | CH₂CH₂CH₃ |
| I-112 | (CH₂)₂-O-CH₂CH₂CH₂CH₂CH₂CH₃ | CH₂CH₂CH₃ |
| I-113 | (CH₂)₂-O-CH₂CH₂CH₂CH₂CH₂CH₂CH₃ | CH₂CH₂CH₃ |
| I-114 | (CH₂)₂-O-CH₂CH₂CH₂CH₂CH₂CH₂CH₂CH₃ | CH₂CH₂CH₃ |
| I-115 | (CH₂)₂-O-(CH₂)₈CH₃ | CH₂CH₂CH₃ |
| I-116 | (CH₂)₂-O-CH(CH₃)₂ | CH₂CH₂CH₃ |
| I-117 | (CH₂)₂-O-C(CH₃)₃ | CH₂CH₂CH₃ |
| I-118 | (CH₂)₂-O-CH₂C(CH₃)₃ | CH₂CH₂CH₃ |
| I-119 | (CH₂)₂-O-CH(CH₃)CH₂C(CH₃)₃ | CH₂CH₂CH₃ |
| I-120 | (CH₂)₂-O-CH(CH₂CH₃)CH₂C(CH₃)₃ | CH₂CH₂CH₃ |
| I-121 | (CH₂)₂-O-CH₂CH(CH₃)CH₂CH(CH₃)₂ | CH₂CH₂CH₃ |
| I-122 | (CH₂)₂-O-CH₂CH(CH₂CH₃)CH₂CH₂CH₃ | CH₂CH₂CH₃ |
| I-123 | (CH₂)₂-O-CH₂CH₂CH(CH₃)CH₂CH(CH₃)₂ | CH₂CH₂CH₃ |
| I-124 | (CH₂)₂-O-CH₂CH₂CH(CH₃)CH₂C(CH₃)₃ | CH₂CH₂CH₃ |
| I-125 | (CH₂)₂-O-CH₂CH₂CH(CH₃)CH₂CH₂CH(CH₃)₂ | CH₂CH₂CH₃ |
| I-126 | (CH₂)₃-O-CH₂CH₂CH(CH₃)(CH₂)₃CH(CH₃)₂ | CH₂CH₂CH₃ |
| I-127 | (CH₂)₃-O-CH₃ | CH₂CH₂CH₃ |
| I-128 | (CH₂)₃-O-CH₂CH₃ | CH₂CH₂CH₃ |
| I-129 | (CH₂)₃-O-CH₂CH₂CH₃ | CH₂CH₂CH₃ |
| I-130 | (CH₂)₃-O-CH₂CH₂CH₂CH₃ | CH₂CH₂CH₃ |
| I-131 | (CH₂)₃-O-CH₂CH₂CH₂CH₂CH₃ | CH₂CH₂CH₃ |
| I-132 | (CH₂)₃-O-CH₂CH₂CH₂CH₂CH₂CH₃ | CH₂CH₂CH₃ |
| I-133 | (CH₂)₃-O-CH₂CH₂CH₂CH₂CH₂CH₂CH₃ | CH₂CH₂CH₃ |
| I-134 | (CH₂)₃-O-CH₂CH₂CH₂CH₂CH₂CH₂CH₂CH₃ | CH₂CH₂CH₃ |
| I-135 | (CH₂)₃-O-(CH₃)₈CH₃ | CH₂CH₂CH₃ |
| I-136 | (CH₂)₃-O-CH(CH₃)₂ | CH₂CH₂CH₃ |
| I-137 | (CH₂)₃-O-C(CH₃)₃ | CH₂CH₂CH₃ |
| I-138 | (CH₂)₃-O-CH₂C(CH₃)₃ | CH₂CH₂CH₃ |
| I-139 | (CH₂)₃-O-CH(CH₃)CH₂C(CH₃)₃ | CH₂CH₂CH₃ |
| I-140 | (CH₂)₃-O-CH(CH₂CH₃)CH₂C(CH₃)₃ | CH₂CH₂CH₃ |
| I-141 | (CH₂)₃-O-CH₂CH(CH₃)CH₂CH(CH₃)₂ | CH₂CH₂CH₃ |
| I-142 | (CH₂)₃-O-CH₂CH(CH₂CH₃)CH₂CH₂CH₃ | CH₂CH₂CH₃ |
| I-143 | (CH₂)₃-O-CH₂CH₂CH(CH₃)CH₂CH(CH₃)₂ | CH₂CH₂CH₃ |
| I-144 | (CH₂)₃-O-CH₂CH₂CH(CH₃)CH₂C(CH₃)₃ | CH₂CH₂CH₃ |
| I-145 | (CH₂)₃-O-CH₂CH₂CH(CH₃)CH₂CH₂CH(CH₃)₂ | CH₂CH₂CH₃ |
| I-146 | (CH₂)₃-O-CH₂CH₂CH(CH₃)(CH₂)₃CH(CH₃)₂ | CH₂CH₂CH₃ |
| I-147 | CH₂CH₂CH₃ | CH₂OCH₃* |
| I-148 | CH₂CH₂CH₂CH₃ | CH₂OCH₃* |
| I-149 | CH₂CH₂CH₂CH₂CH₃ | CH₂OCH₃* |
| I-150 | CH₂CH₂CH₂CH₂CH₂CH₃ | CH₂OCH₃* |
| I-151 | CH₂CH(CH₃)CH₂CH₂CH₃ | CH₂OCH₃* |
| I-152 | CH₂CH(CH₂CH₃)₂ | CH₂OCH₃* |
| I-153 | CH₂CH₂CH₂CH_{I}CH₂CH₂CH₃ | CH₂OCH₃* |
| I-154 | CH₂CH₂CH₂CH₂CH₂CH₂CH₂CH₃ | CH₂OCH₃* |
| I-155 | CH₂CH₂CH₂CH₂CH₂CH₂CH₂CH₂CH₃ | CH₂OCH₃* |
| I-156 | CH₂CH₂CH₂CH₂CH₂CH₂CH₂CH₂CH₂CH₃ | CH₂OCH₃* |
| I-157 | CH₂CH₂CH(CH₃)CH₂C(CH₃)₃ | CH₂OCH₃* |
| I-158 | CH₂CH₂C(CH₃)₂CH₂C(CH₃)₃ | CH₂OCH₃* |
| I-159 | (CH₂)₃-O-CH₃ | CH₂OCH₃* |
| I-160 | (CH₂)₃-O-CH₂CH₃ | CH₂OCH₃* |
| I-161 | (CH₂)₃-O-CH₂CH₂CH₃ | CH₂OCH₃* |
| I-162 | (CH₂)₃-O-CH₂CH₂CH₂CH₃ | CH₂OCH₃* |
| I-163 | (CH₂)₃-O-CH₂CH₂CH₂CH₂CH₃ | CH₂OCH₃* |
| I-164 | (CH₂)₃-O-CH₂CH₂CH₂CH₂CH₂CH₃ | CH₂OCH₃* |
| I-165 | (CH₂)₃-O-CH₂CH₂CH₂CH₂CH₂CH₂CH₃ | CH₂OCH₃* |
| I-166 | (CH₂)₃-O-CH₂CH₂CH₂CH₂CH₂CH₂CH₂CH₃ | CH₂OCH₃* |
| I-167 | (CH₂)₃-O-(CH₃)₈CH₃ | CH₂OCH₃* |
| I-168 | (CH₂)₃-O-CH(CH₃)₂ | CH₂OCH₃* |
| I-169 | (CH₂)₃-O-C(CH₃)₃ | CH₂OCH₃* |
| I-170 | (CH₂)₃-O-CH₂C(CH₃)₃ | CH₂OCH₃* |
| I-171 | (CH₂)₃-O-CH(CH₃)CH₂C(CH₃)₃ | CH₂OCH₃* |
| I-172 | (CH₂)₃-O-CH(CH₂CH₃)CH₂C(CH₃)₃ | CH₂OCH₃* |
| I-173 | (CH₂)₃-O-CH₂CH(CH₃)CH₂CH(CH₃)₂ | CH₂OCH₃* |
| I-174 | (CH₂)₃-O-CH₂CH(CH₂CH₃)CH₂CH₂CH₃ | CH₂OCH₃* |
| I-175 | (CH₂)₃-O-CH₂CH₂CH(CH₃)CH₂CH(CH₃)₂ | CH₂OCH₃* |
| I-176 | (CH2)₃-O-CH₂CH₂CH(CH₃)CH₂C(CH₃)₃ | CH₂OCH₃* |
| I-177 | (CH₂)₃-O-CH₂CH₂CH(CH₃)CH₂CH₂CH(CH₃)₂ | CH₂OCH₃* |
| I-178 | (CH₂)₃-O-CH₂CH₂CH(CH₃)(CH₂)₃CH(CH₃)₂ | CH₂OCH₃* |
| I-179 | CH₃* | (CH₂)₃CH₃ |
| I-180 | CH₂CH₃* | (CH₂)₃CH₃ |
| I-181 | CH₂CH₂CH₃ | (CH₂)₃CH₃ |
| I-182 | CH₂CH₂CH₂CH₃ | (CH₂)₃CH₃ |
| I-183 | CH₂CH₂CH₂CH₂CH₃ | (CH₂)₃CH₃ |
| I-184 | CH₃* | (CH₂)₄CH₃ |
| I-185 | CH₂CH₃* | (CH₂)₄CH₃ |
| I-186 | CH₂CH₂CH₃ | (CH₂)₄CH₃ |
| I-187 | CH₂CH₂CH₂CH₃ | (CH₂)₄CH₃ |
| I-188 | CH₂CH₂CH₂CH₂CH₃ | (CH₂)₄CH₃ |
| I-189 | CH₃* | (CH₂)₅CH₃ |
| I-190 | CH₂CH₃* | (CH₂)₅CH₃ |
| I-191 | CH₂CH₂CH₃ | (CH₂)₅CH₃ |
| I-192 | CH₂CH₂CH₂CH₃ | (CH₂)₅CH₃ |
| I-193 | CH₂CH₂CH₂CH₂CH₃ | (CH₂)₅CH₃ |
| I-194 | CH₃* | (CH₂)₆CH₃ |
| I-195 | CH₂CH₃* | (CH₂)₆CH₃ |
| I-196 | CH₂CH₂CH₃ | (CH₂)₆CH₃ |
| I-197 | CH₂CH₂CH₂CH₃ | (CH₂)₆CH₃ |
| I-198 | CH₂CH₂CH₂CH₂CH₃ | (CH₂)₆CH₃ |
| I-199 | CH₃* | (CH₂)₇CH₃ |
| I-200 | CH₂CH₃* | (CH₂)₇CH₃ |
| I-201 | CH₂CH₂CH₃ | (CH₂)₇CH₃ |
| I-202 | CH₂CH₂CH₂CH₃ | (CH₂)₇CH₃ |
| I-203 | CH₂CH₂CH₂CH₂CH₃ | (CH₂)₇CH₃ |
| I-204 | CH₃* | (CH₂)₈CH₃* |
| I-205 | CH₂CH₃* | (CH₂)₈CH₃* |
| I-206 | CH₂CH₂CH₃ | (CH₂)₈CH₃* |
| I-207 | CH₂CH₂CH₂CH₃ | (CH₂)₈CH₃* |
| I-208 | CH₂CH₂CH₂CH₂CH₃ | (CH₂)₈CH₃* |
| I-209 | CH₂CH₃* | CH₂CH₂CH₂CH₂CH₂CH₃ |
| I-210 | CH₂CH₃* | CH₂CH(CH₃)CH₂CH₂CH₃ |
| I-211 | CH₂CH₃* | CH₂CH₂CH₂CH₂CH₂CH₂CH₃ |
| I-212 | CH₂CH₃* | CH₂CH₂CH₂CH₂CH₂CH₂CH₂C H₃ |
| I-213 | CH₂CH₃* | CH₂CH₂CH(CH₃)CH₂C(CH₃)₃* |
| I-214 | CH₂CH₃* | CH₂CH₂C(CH₃)₂CH₂C(CH₃)₃* |
| I-215 | CH₂CH₃* | CH₂CH₂CH₂CH₂CH₂CH₃ |
| I-216 | CH₂CH₃* | CH₂CH(CH₃)CH₂CH₂CH₃ |
| I-217 | CH₂CH=CH₂ | CH₃ |
| I-218 | CH₂CH=CH₂ | CF₃* |
| I-219 | CH₂CH=CH₂ | CH₂CH₃ |
| I-220 | CH₂CH=CH₂ | CH₂CH₂CH₃ |
| I-221 | CH₂CH=CH₂ | CH₂OCH₃* |
| I-222 | CH₂CH=CH₂ | (CH₂)₃CH₃ |
| I-223 | CH₂CH=CH₂ | (CH₂)₄CH₃ |
| I-224 | CH₂CH=CH₂ | (CH₂)₅CH₃ |
| I-225 | CH₂CH=CH₂ | (CH₂)₆CH₃ |
| I-226 | CH₂CH=CH₂ | (CH₂)₇CH₃ |
| I-227 | CH₂CH=CH₂ | (CH₂)₈CH₃* |

| | | |
|---|---|---|
| ^{*)} nicht erfindungsgemäßes Beispiel | | |

Als Beispiele für geeignete Nitrile der Formel II sind solche Verbindungen zu nennen, worin R¹ eine der in den Zeilen I-1 bis I-35 angegebenen Bedeutungen aufweist. Als Beispiele für geeignete Nitrile der Formel II sind weiterhin solche Verbindungen zu nennen, worin R¹ eine der in den Zeilen I-109 bis I-126, I-180 und I-217 angegebenen Bedeutungen aufweist.

Für das erfindungsgemäße Verfahren hat es sich weiterhin als vorteilhaft erwiesen, wenn R³ in Formel III für C₁-C₈-Alkyl oder C₁-C₄-Alkoxy-C₁-C₄-alkyl steht. Insbesondere steht R³ in Formel III für C₁-C₄-Alkyl, besonders bevorzugt für lineares C₁-C₄-Alkyl und speziell für Methyl oder Ethyl.

Beispiele für geeignete Carbonsäureester der Formel III sind die in der Tabelle 2 angegebenen Verbindungen III-1 bis III-30, worin R² und R³ jeweils die in einer Zeile der Tabelle angegebenen Bedeutungen aufweisen:

**Tabelle 2:**

| | R² | R³ |
|---|---|---|
| III-1 | CH₃ | CH₃ |
| III-2 | C₂H₅ | CH₃ |
| III-3 | CF₃* | CH₃ |
| III-4 | CH₂-OCH₃* | CH₃ |
| III-5 | CH₂CH₂CH₃ | CH₃ |
| III-6 | (CH₂)₃CH₃ | CH₃ |
| III-7 | (CH₂)₄CH₃ | CH₃ |
| III-8 | (CH₂)₅CH₃ | CH₃ |
| III-9 | (CH₂)₆CH₃ | CH₃ |
| III-10 | (CH₂)₇CH₃ | CH₃ |
| III-11 | (CH₂)₅CH₃* | CH₃ |
| III-12 | CH₂CH(CH₃)CH₂CH₂CH₃ | CH₃ |
| III-13 | CH₂CH₂CH(CH₃)CH₂C(CH₃)₃* | CH₃ |
| III-14 | CH₂CH₂C(CH₃)₂CH₂C(CH₃)₃* | CH₃ |
| III-15 | CH₂CH(CH₃)CH₂CH₂CH₃ | CH₃ |
| III-16 | CH₃ | CH₂CH₃ |
| III-17 | C₂H₅ | CH₂CH₃ |
| III-18 | CF₃* | CH₂CH₃ |
| III-19 | CH₂-OCH₃* | CH₂CH₃ |
| III-20 | CH₂CH₂CH₃ | CH₂CH₃ |
| III-21 | (CH₂)₃CH₃ | CH₂CH₃ |
| III-22 | (CH₂)₄CH₃ | CH₂CH₃ |
| III-23 | (CH₂)₅CH₃ | CH₂CH₃ |
| III-24 | (CH₂)₆CH₃ | CH₂CH₃ |
| III-25 | (CH₂)₇CH₃ | CH₂CH₃ |
| III-26 | (CH₂)₈CH₃* | CH₂CH₃ |
| III-27 | CH₂CH(CH₃)CH₂CH₂CH₃ | CH₂CH₃ |
| III-28 | CH₂CH₂CH(CH₃)CH₂C(CH₃)₃* | CH₂CH₃ |
| III-29 | CH₂CH₂C(CH₃)₂CH₂C(CH₃)₃* | CH₂CH₃ |
| III-30 | CH₂CH(CH₃)CH₂CH₂CH₃ | CH₂CH₃ |

| | | |
|---|---|---|
| ^{*)} nicht erfindungsgemäßes Beispiel | | |

Die erfindungsgemäß erhältlichen ß-Ketonitrile können in besonders vorteilhafter Weise zur Herstellung von 7-Aminoazolopyrimidinen der allgemeinen Formel IV eingesetzt werden.

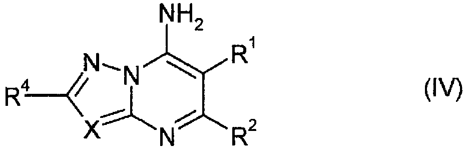

In Formel IV haben R¹ und R² die zuvor genannten Bedeutungen. R⁴ steht für Wasserstoff, NH₂ oder C₁-C₆-Alkyl. X steht für N oder eine Gruppe C-R⁵, worin R⁵ für Wasserstoff oder C₁-C₆-Alkyl steht. Gemäß einer bevorzugten Ausführungsform der Erfindung steht X für N. Gemäß einer anderen bevorzugten Ausführungsform der Erfindung steht X für CH.

Die Herstellung der 7-Aminoazolopyrimidine der allgemeinen Formel IV kann in Analogie zu bekannten Verfahren des Standes der Technik, wie sie beispielsweise in EP-A 141317 und WO2006/087325 beschrieben sind, erfolgen, z. B. durch Umsetzung von ß-Ketonitrilen der allgemeinen Formel I mit einer Aminoazol-Verbindung der Formel V 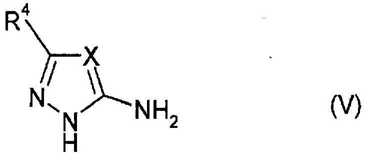 worin R⁴ und X die zuvor genannten Bedeutungen aufweisen, oder einem Tautomer von V.

Dementsprechend betrifft die vorliegende Erfindung auch ein Verfahren zur Herstellung von 7-Aminoazolopyrimidinen der allgemeinen Formel IV, welches die folgenden Verfahrensschritte umfasst:
a) Herstellung eines ß-Ketonitrils der allgemeinen Formel I nach dem hier beschriebenen Verfahren und
b) Umsetzung des ß-Ketonitrils der Formel I mit einer Aminoazol-Verbindung der Formel V oder einem Tautomer davon in an sich bekannter Weise, wobei man das Azolopyrimidin der Formel IV erhält.

Die Umsetzung des ß-Ketonitrils der Formel I mit der Aminoazol-Verbindung der Formel V bzw. mit ihrem Tautomeren erfolgt vorzugsweise in Gegenwart einer Säure. Als Säuren sind grundsätzlich Carbonsäuren aber auch organische Sulfonsäuren geeignet. Im Falle der Sulfonsäuren werden typischerweise katalytische Mengen eingesetzt, die sich üblicherweise im Bereich von 1 bis 40 Mol-%, bezogen auf ein Mol Aminoazol der Formel V, bewegen. Im Falle der Carbonsäuren können diese auch als Lösungsmittel fungieren.

Beispiele für geeignete organische Sulfonsäuren sind Methansulfonsäure, Trifluormethansulfonsäure, Benzolsulfonsäure, 2,3-Dimethylbenzolsufonsäure, 3,4-Dimethylbenzolsulfonsäure und p-Toluolsulfonsäure. Geeignete organische Carbonsäuren sind Ameisensäure, Essigsäure, Propionsäure, 2-Methylpropionsäure, Benzoesäure und deren Gemische.

Vorzugsweise erfolgt die Umsetzung in einem organischen, inerten Lösungsmittel. Bevorzugt sind solche organischen Lösungsmittel, in denen die Einsatzstoffe wenigstens teilweise oder vollständig löslich sind. Beispiele für geeignete Lösungsmittel sind insbesondere C₁-C₄-Alkanole wie Methanol, Ethanol, Propanol, Isopropanol, n-Butanol, Isobutanol, tert.-Butanol, die vorgenannten acyclischen und alicyclischen Ether, aromatische Kohlenwasserstoffe, insbesondere Alkylaromaten wie zuvor genannt sowie Halogenaromaten, z. B. Chlorbenzol, Dichlorbenzol, weiterhin Glykole und Glykol-Monoalkylether, Diethylenglykol und deren Monoalkylether, Amide und Lactame, insbesondere N,N-Di-C₁-C₄-Alkylamide aliphatischer Carbonsäuren mit 1 bis 4 C-Atomen wie Dimethylformamid, Diethylformamid, Dibutylformamid, N,N-Dimethylacetamid, die vorgenannten Carbonsäuren und Gemische dieser Lösungsmittel sowie Gemische dieser Lösungsmittel mit Wasser. In einer bevorzugten Ausführungsform besteht das inerte Lösungsmittel zu wenigstens 80 Gew.-% und insbesondere zu wenigstens 90 Gew.-% aus aromatischen Lösungsmitteln, insbesondere aus Alkylaromaten.

Die Umsetzung von IV mit I erfolgt vorzugsweise bei Temperaturen im Bereich von 80 bis 250 °C, insbesondere im Bereich von 120 bis 220 °C und speziell im Bereich von 150 bis 190 °C.

Vorteilhafterweise geht man bei der Umsetzung so vor, dass man das bei der Reaktion gebildete Wasser entfernt, gegebenenfalls abdestilliert, z. B. als Azeotrop mit dem eingesetzten Lösungsmittel.

In einer bevorzugten Ausführungsform der Erfindung werden solche Aminoazole V eingesetzt, worin X für N steht. In einer anderen, ebenfalls bevorzugten Ausführungsform werden Aminoazole der Formel V eingesetzt, worin X für CH steht. In den Formeln IV und V steht R⁴ vorzugsweise für Wasserstoff.

Beispiele für bevorzugte Aminoazole V sind 3-Amino-2H-1,2,4-triazol und dessen Tautomere 3-Amino-1H-1,2,4-triazol und 3-Amino-4H-1,3,4-triazol sowie 3-Amino-1H-pyrazol und dessen Tautomer 3-Amino-2H-pyraxol.

Gemäß einer bevorzugten Ausführungsform erfolgt Schritt b) unmittelbar im Anschluss an Schritt a), ohne dass eine Isolierung des ß-Ketonitrils notwendig ist. Insbesondere setzt man in Schritt b) des Verfahrens eine Lösung des ß-Ketonitrils I in einem inerten organischen Lösungsmittel ein, die nach Neutralisation des in Schritt a) gebildeten Kaliumsalzes des ß-Ketonitrils der Formel I erhalten wurde. Insbesondere handelt es sich um eine Lösung in einem inerten Lösungsmittel, das zu wenigstens 80 Gew.-% und insbesondere wenigstens 90 Gew.-% aus einem oder mehren Alkylaromaten besteht.

Die Umsetzung des Aminoazols IV oder seines Tautomeren kann sowohl absatzweise als auch kontinuierlich erfolgen. Üblicherweise erfolgt sie absatzweise. Hierzu geht man in der Regel so vor, dass man das Aminoazol IV und das ß-Ketonitril in einem Reaktionsgefäß gegebenenfalls zusammen mit Lösungsmittel und gegebenenfalls Säure vorlegt und das Gemisch auf Reaktionstemperatur erhitzt. Gegebenenfalls destilliert man dabei einen Teil des Lösungsmittels zusammen mit dem gebildeten Reaktionswasser ab. Als Reaktionsgefäße kommen die für Schritt a) genannten Reaktoren in Betracht, die gegebenenfalls noch mit Vorrichtungen zum Abdestillieren von Lösungsmitteln ausgestattet sein können.

Aus dem so erhaltenen Reaktionsgemisch kann man die Verbindung IV in üblicher Weise gewinnen, beispielsweise durch eine wässrige Aufarbeitung, gegebenenfalls gefolgt von einer Kristallisation zur Reinigung oder durch Entfernen des Lösungsmittels und anschließendes Umkristallisieren des Produkts.

Die folgenden Beispiele dienen der Erläuterung der Erfindung.

Vergleichsbeispiel 1 (Beispiel 1 0 der US2004/0171863)

In einem Stahlautoklaven mit einem Reaktionsvolumen von 250 ml wurden 10,88 g Natriummethylat (0,20 Mol), 41,70 g (0,60 Mol) n-Butyronitril und 30,14 g (0,43 Mol) n-Butylacetat bei Raumtemperatur vorgelegt. Man spülte den Autoklaven mit Stickstoff, verschloss den Autoklaven und erhitzte zwei Stunden auf 150 °C. Der nach dem Abkühlen erhaltene Feststoff wurde abfiltriert und getrocknet. Man erhielt auf diese Weise 16,9 g 3-Cyanobutan-2-on in Form seines Natriumsalzes (Rohprodukt).

16,7 g des Rohprodukts wurden in das Reaktionsgefäß gegeben. Anschließend gab man 20 ml entionisiertes Wasser und 50 ml Ethylacetat zu und rührte. Danach säuerte man mit insgesamt 11 ml konzentrierter Salzsäure an. Der pH-Wert der wässrigen Phase betrug danach pH 5. Die organische Phase wurde abgetrennt und über Magnesiumsulfat getrocknet. Nach Filtration wurde die organische Lösung mit Rotationsverdampfer zur Trockne eingeengt. Man erhielt auf diese Weise 9,00 g einer orangefarbenen klaren Flüssigkeit, die laut Gaschromatogramm zu 88,8 % aus 3-Cyanopentanon bestand. Dies entspricht einer Absolutausbeute von 7,99 g bzw. 27,7 %, bezogen auf eingesetztes n-Butylacetat.

### Vergleichsbeispiel 2

In einem Stahlautoklaven mit einem Reaktionsvolumen von 250 ml wurden 7,34 g Natriummethylat, 48,5 g Xylol, 64 g (0,40 Mol) Decannitril und 17,9 g (0,17 Mol) Propionsäureethylester vorgelegt. Der Autoklav wurde mit Stickstoff gespült, verschlossen und anschließend zwei Stunden auf 150 °C erhitzt. Das nach dem Abkühlen erhaltene Natriumsalz wurde abfiltriert und getrocknet. Man erhielt auf diese Weise 38,8 g trockenes Rohprodukt, das in einen Glaskolben gefüllt, mit 27,00 g entionisiertem Wasser und 60,84 g Ethylacetat versetzt wurde. Anschließend säuerte man mit 14,50 ml konzentrierter Salzsäure an. Der pH-Wert der wässrigen Phase nach Salzsäurezugabe lag bei pH 4,5. Die organische Phase wurde abgetrennt, über Magnesiumsulfat getrocknet, filtriert und anschließend am Rotationsverdampfer zur Trockne eingeengt. Man erhielt auf diese Weise 62,80 g eines Rohprodukts, das laut Gaschromatogramm zu 78,2 % aus unumgesetztem Nitril und zu 11,27 % aus dem gewünschten Produkt bestand.

### Erfindungsgemäße Beispiele

### Beispiel 1: 2-(2-Methoxyacetyl)-decanonitril (nicht erfindungsgemäß)

In einem Rührgefäß mit Rückflusskühler und Tropftrichter wurden 199,96 g festes Kaliummethylat vorgelegt und mit 318 g o-Xylol versetzt. Man fügte noch 15 g Ethanol zu und erhitzte die Suspension auf Rückfluss. Sodann wurde über den Tropftrichter ein Gemisch aus 170 g Decanonitril und 180,8 g Methoxyessigsäureethylester innerhalb von 10 min. zugegeben. Nach Beendigung der Zugabe rührte man noch 6 h nach und kühlte dann auf 105 °C ab. Nun fügte man 373 g Wasser zu und stellte die Mischung durch Zugabe von ca. 388 g 20%iger Salzsäure auf einen pH-Wert von 4 ein. Man temperierte auf 70 °C und trennte die (untere) wässrige Phase ab. Die organische Phase wurde nochmals mit 186 g Wasser gewaschen. Nach Abtrennen der wässrigen Phase wurde die organische Phase zur Wasserabtrennung auf die Hälfte eingeengt. Es verblieben 235,5 g einer blassgelben Lösung mit einem Gehalt an Wertprodukt von 62%-a/a (GC), die so weiter umgesetzt werden kann. Zur Reinigung wurde die Verbindung im Hochvakuum destilliert, wobei das Produkt bei 120 bis 134 °C/1mbar überging.
¹H-NMR (CDCl₃): 0,89(t, 3H); 1,2-1,55(m, 12H); 1,88(m, 2H); 3,45(s, 3H), 3,75 (dd, 1H); 4,20(s, 2H).

### Beispiel 2: 2-Acetyl-decanonitril

In einem Rührgefäß mit Rückflusskühler und Tropftrichter wurden 317 g o-Xylol vorgelegt, mit 198,96 g festem Kaliummethylat versetzt und auf Rückfluss erhitzt. Sodann wurde über den Tropftrichter ein Gemisch aus 170 g Decanonitril und 133,9 g Essigsäureethylester innerhalb von 10 min. zugegeben. Nach Beendigung der Zugabe rührte man noch 4 h nach und kühlte dann auf 105 °C ab. Nun fügte man 372 g Wasser zu und stellte die Mischung durch Zugabe von ca. 470 g 20%iger Salzsäure auf einen pH-Wert von 4,3 ein. Man temperierte auf 65 °C und trennte die (untere) wässrige Phase ab. Die organische Phase wurde nochmals mit 186 g Wasser gewaschen. Nach Abtrennen der wässrigen Phase wurde die organische Phase zur Wasserabtrennung auf ca. die Hälfte eingeengt. Es verblieben 211,7 g einer hellgelben Lösung mit einem Gehalt an Wertprodukt von 66,8%-a/a (GC), die so weiter umgesetzt werden kann. Zur Reinigung wurde die Verbindung im Hochvakuum destilliert, wobei das Produkt bei 106 bis 121 °C/1mbar überging.
¹H-NMR: 0,9(t, 3H); 1,1-1,7(m, 12H); 1,9(m, 2H); 2,38(s, 3H); 3,40(dd, 1H).

### Beispiel 3: 2-Propionylpent-4-en-nitril

In einem 20 l-Doppelmantelreaktor mit Heiz-/Kühlkreislauf und Rückflusskühler wurden 3500 g Kalium-tert.-butylat vorgelegt und mit 6316 g Tetrahydrofuran versetzt. Bei einer Temperatur von maximal 10 °C wurde nun ein Gemisch aus 1200 g Pentennitril und 1651 g Propionsäureethylester innerhalb von 75 min zugepumpt. Man rührte noch 3 h bei 8 bis 10 °C und dann über Nacht bei 15 °C nach. Nun stellte man die Mischung durch Zupumpen von 6316 g 20%iger Salzsäure auf einen pH-Wert von 3,8 ein wobei die Temperatur auf 48 °C stieg. Nun wurden 2400 ml Wasser und 4000 ml o-Xylol zugegeben. Die wässrige (untere) Phase wurde abgetrennt und die organische Phase wurde noch zweimal mit je 2400 ml Wasser gewaschen. Es verblieben 14650 g organische Phase. Einengen am Rotationsverdampfer bei 60 °C und 10mbar lieferte 1873 g bernsteinfarbenes Produkt mit einem Gehalt von 87,4%-a/a (GC), die so weiter umgesetzt wurden.
¹H-NMR: 1,12(s, 3H); 2,52(q) + 2,75(m) (zus. 4H); 3,50(t, 1 H); 5,25(m, 2H); 5,80(m, 1 H).

### Beispiel 4: 2-Propionyl-decanonitril

In einem Rührgefäß mit Rückflusskühler und Tropftrichter wurden 724 g o-Xylol vorgelegt, mit 454,2 g festem Kaliummethylat versetzt und auf Rückfluss erhitzt. Über den Tropftrichter wurden nun zunächst 10 % eines Gemisches aus 392,0 g Decanonitril und 360,6 g Propionsäureethylester innerhalb von 1 min zugegeben und der Ansatz wurde 15 min gerührt. Sodann wurde die Restmenge des Gemischs innerhalb von 10 min zudosiert. Nach Beendigung der Zugabe rührte man noch 4 h unter schwachem Rückfluss nach und kühlte dann auf 105 °C ab. Die Mischung wurde auf 848 g Wasser in einen zweiten Kessel abgelassen durch Zugabe von ca. 1020 g 20%iger Salzsäure auf einen pH-Wert von 6 eingestellt. Man temperierte auf 80 °C und trennte die (untere) wässrige Phase ab. Es verblieben 1316 g einer hellgelben Lösung mit einem Gehalt an Wertprodukt von 37,6 %-a/a (GC), die so weiter umgesetzt werden kann. Zur Reinigung wurde die Verbindung im Hochvakuum destilliert, wobei das Produkt bei 130 bis 140 °C/1mbar überging. ¹H-NMR: 0,9(t, 3H); 1,11(t, 3H), 1,2-1,4(m, 10H); 1,4-1,55(m, 2H); 1,8-2,0(m, 2H); 2,75(q, 2H), 3,40(t, 1H).

Allgemeine Vorschrift A zur Herstellung von ß Ketonitrilen der allgemeinen Formel I:

In einem Reaktionsgefäß mit Rührer, Rückflusskühler und Stickstoffzufuhr legt man 587 g (7,96 Mol) Kaliummethylat, 940 g ortho-Xylol und 25 g Ethanol vor und erhitzt anschließend unter Stickstoff unter Rückfluss (Badtemperatur 150 °C, Innentemperatur 132 °C). Anschließend gibt man über zwei getrennte Zuläufe zeitgleich beginnend innerhalb 21 Minuten 3,23 Mol Nitril der Formel I und 4,4 Mol Ester der Formel II in das Reaktionsgefäß unter Beibehaltung der Temperatur. Anschließend erhitzt man weitere vier Stunden zum Rückfluss und kühlt danach auf 105 °C ab. Der noch heiße Inhalt des Reaktionskolbens wird in einen Reaktionskessel, der 1190 g Wasser enthält, abgelassen. Die erhaltene Mischung wird gerührt, anschließend mit 1350 g 20 gew.-%iger wässriger Salzsäure auf pH 4-5 eingestellt und weitere 15 min. gerührt. Die wässrige Phase wird mit 20 gew.-%iger Salzsäure auf pH 4 bis 5 eingestellt und dann 15 Minuten gerührt. Anschließend trennt man die wässrige Phase ab und wäscht die organische Phase mit insgesamt 1100 g entionisiertem Wasser. Die organische Phase wird anschließend am Rotationsverdampfer auf etwa 75 % ihres Ausgangsvolumens eingeengt.

Zur Reinigung der Verbindung wird zunächst unter reduziertem Druck das Lösungsmittel abdestilliert. Der verbleibende Rückstand wird dann im Hochvakuum rektifiziert. Die Verbindungen der Formel I fallen dabei als Hauptfraktion in Form farbloser bis blassgelber Öle an. Als Vorfraktionen erhält man den nicht umgesetzten Teil der Edukte, die wieder eingesetzt werden können.

Auf diese Weise können die folgenden Verbindungen der Formel I, soweit nichts anderes angegeben ist, hergestellt werden:

| Bsp. | R¹ | R² | Ausbeute [%]¹⁾ |
|---|---|---|---|
| 1 | CH₂CH₂CH₂CH₂CH₂CH₂CH₂CH₃ | CH₂OCH₃* | 59,2 |
| 2 | CH₂CH₂CH₂CH₂CH₂CH₂CH₃ | CH₃ | 66,1 |
| 3 | CH₂CH=CH₂ | CH₂CH₃ | 81,0 |
| 4 | CH₂CH₂CH₂CH₂CH₂CH₂CH₂CH₃ | CH₂CH₃ | 92,0 |
| 5 | CH₂CH₂CH(CH₃)CH₂C(CH₃)₃ | CH₃ | |
| 6 | CH₂CH₂C(CH₃)₂CH₂C(CH₃)₃ | CH₃ | |
| 7 | CH₂CH₂CH₂CH₂CH₂CH₃ | CF₃* | |
| 8 | CH₂CH(CH₃)CH₂CH₂CH₃ | CF₃* | |
| 9 | CH₂CH₂CH₂CH₂CH₂CH₂CH₃ | CF₃* | |
| 10 | CH₂CH₂CH₂CH₂CH₂CH₂CH₂CH₃ | CF₃* | |
| 11 | CH₂CH₂CH(CH₃)CH₂C(CH₃)₃ | CF₃* | |
| 12 | CH₂CH₂C(CH₃)₂CH₂C(CH₃)₃ | CF₃* | |
| 13 | CH₂CH₂CH₂CH₂CH₂CH₃ | CH₂CH₃ | |
| 14 | CH₂CH(CH₃)CH₂CH₂CH₃ | CH₂CH₃ | |
| 15 | CH₂CH₂CH₂CH₂CH₂CH₂CH₃ | CH₂CH₃ | |
| 16 | CH₂CH₂CH₂CH₂CH₂CH₂CH₂CH₃ | CH₃ | |
| 17 | CH₂CH₂CH(CH₃)CH₂C(CH₃)₃ | CH₂CH₃ | |
| 18 | CH₂CH₂C(CH₃)₂CH₂C(CH₃)₃ | CH₂CH₃ | |
| 19 | CH₂CH₂CH₂CH₂CH₂CH₃ | CH₂CH₂CH₃ | |
| 20 | CH₂CH(CH₃)CH₂CH₂CH₃ | CH₂CH₂CH₃ | |
| 21 | CH₂CH₂CH₂CH₂CH₂CH₂CH₃ | CH₂CH₂CH₃ | |
| 22 | CH₂CH₂CH₂CH₂CH₂CH₂CH₂CH₃ | CH₂CH₂CH₃ | |
| 23 | CH₂CH₂CH(CH₃)CH₂C(CH₃)₃ | CH₂CH₂CH₃ | |
| 24 | CH₂CH₂C(CH₃)₂CH₂C(CH₃)₃ | CH₂CH₂CH₃ | |
| 25 | CH₂CH₂CH₂CH₂CH₂CH₃ | CH₂OCH₃* | |
| 26 | CH₂CH(CH₃)CH₂CH₂CH₃ | CH₂OCH₃* | |
| 27 | CH₂CH₂CH₂CH₂CH₂CH₂CH₃ | CH₂OCH₃* | |
| 28 | CH₂CH₂CH₂CH₂CH₂CH₃ | CH₃ | |
| 29 | CH₂CH₂CH(CH₃)CH₂C(CH₃)₃ | CH₂OCH₃* | |
| 30 | CH₂CH₂C(CH₃)₂CH₂C(CH₃)₃ | CH₂OCH₃* | |
| 31 | CH₂CH₃* | CH₂CH₂CH₂CH₂CH₂CH₃ | |
| 32 | CH₂CH₃* | CH₂CH(CH₃)CH₂CH₂CH₃ | |
| 33 | CH₂CH₃* | CH₂CH₂CH₂CH₂CH₂CH₂CH₃ | |
| 34 | CH₂CH₃* | CH₂CH₂CH₂CH₂CH₂CH₂CH₂CH₃ | |
| 35 | CH₂CH₃* | CH₂CH₂CH(CH₃)CH₂C(CH₃)₃* | |
| 36 | CH₂CH₃* | CH₂CH₂C(CH₃)₂CH₂C(CH₃)₃* | |
| 37 | CH₂CH=CH₂ | CH₃ | |
| 38 | CH₂CH=CH₂ | CF₃* | |
| 39 | CH₂CH(CH₃)CH₂CH₂CH₃ | CH₃ | |
| 40 | CH₂CH=CH₂ | CH₂CH₂CH₃ | |
| 41 | CH₂CH=CH₂ | CH₂OCH₃* | |
| 42 | CH₂CH=CH₂ | (CH₂)₃CH₃ | |
| 43 | CH₂CH=CH₂ | (CH₂)₄CH₃ | |
| 44 | CH₂CH=CH₂ | (CH₂)₅CH₃ | |
| 45 | CH₂CH=CH₂ | (CH₂)₆CH₃ | |
| 46 | CH₂CH=CH₂ | (CH₂)₇CH₃ | |
| 47 | CH₂CH=CH₂ | (CH₂)₈CH₃* | |

| | | | |
|---|---|---|---|
| ¹⁾ bezogen auf das Nitril II ^{*)} nicht erfindungsgemäßes Beispiel | | | |

### Beispiel 48: 5-Ethyl-6-octyl-[1,2,4]triazolo[1,5-a]pyrimidin-7-ylamin:

In einem 2,5 l-Doppelmantelgefäß mit Kondensator und Wasserabscheider wurden 737 g o-Xylol vorgelegt und mit 39,5 g Chlorsulfonsäure versetzt. Man fügte 191,2 g 3-Amino-1,2,4-triazol und 1254 g einer Lösung von 2-Propionyl-decanonitril (37,7 Gew.-% in o-Xylol) zu und erhitzte auf Rückfluss. Man rührte 12 Stunden unter Rückfluss und trennte dabei das entstandene Reaktionswasser über einen Phasenscheider ab. Als sich kein Wasser mehr abschied, kühlte man auf 140 °C und gab 59,4 g Triethylamin zu. Bei weiterem Abkühlen schied sich das Produkt in Form farbloser Kristalle ab. Bei einer Temperatur von 105 °C gab man 152,8 g Methanol zu. Man kühlt weiter auf 20 °C und trennte den entstandenen Feststoff ab. Der Filterkuchen wurde mit einer Mischung aus 850 g Methanol und 119 g Wasser gewaschen und dann im Vakuum getrocknet. Man erhielt 525,5 g farblose Kristalle mit einem Gehalt von 98,3%-a/a (HPLC). Die Titelverbindung fiel als Gemisch von zwei Modifikationen mit Schmelzpunkten von 199 °C bzw. 201 °C an. Ausbeute: 83,0 %.

### Beispiel 49: 6-Allyl-5-ethyl-[1,2,4]triazolo[1,5-a]pyrimidin-7-ylamin

In einem 20 l-Doppelmantelgefäß mit Rückflusskühler und Wasserabscheider wurden 14650 g o-Xylol vorgelegt und mit 411 g Chlorsulfonsäure versetzt. Man fügte 2074 g 3-Amino-1,2,4-triazol und 3690 g einer Lösung von 2-Propionylpent-4-en-nitril (87,4 % in o-Xylol) zu und erhitzte auf Rückfluss. Man rührte 14 Stunden unter Rückfluss und trennte dabei das entstehende Reaktionswasser über einen Phasenscheider ab. Als sich kein Wasser mehr abschied, kühlte man auf 130 °C und gab 634 g Triethylamin zu. Bei weiterem Abkühlen schied sich das Produkt in Form farbloser Kristalle ab. Bei einer Temperatur von 100 °C gab man 2350 g Isopropanol zu. Man kühlte weiter auf 20 °C und trennte den entstandenen Feststoff ab. Der Filterkuchen wurde mit 1500 ml einer 1:1-Mischung aus Isopropanol und Wasser gewaschen und dann im Vakuum getrocknet. Man erhält 4057 g eines farblosen Pulvers. Schmelzpunkt: 258 °C (Zersetzung), ¹H-NMR: 1,35(t, 3H); 2,88(q, 2H); 3,45(d, 2H); 5,09(d, 2H); 5,20(d, 2H); 5,6(s, breit, 2H); 5,8-6,0(m, 1 H); 8,30(s, 1 H). Ausbeute: 84,0 %.

### Beispiel 50:

Analog Beispiel 48 wurde 5-Methoxymethyl-6-octyl-[1,2,4]triazolo[1,5-a]pyrimidin-7-ylamin ausgehend von 2-(2-Methoxy-acetyl)-decanonitril hergestellt. Man erhielt farblose Kristalle mit einem Schmelzpunkt von 181 bis 182 °C. Ausbeute: 85,1 %.

### Beispiel 51:

Analog Beispiel 48 wurde 5-Methyl-6-octyl-[1,2,4]triazolo[1,5-a]pyrimidin-7-ylamin ausgehend von 2-Acetyl-decanonitril hergestellt. Man erhielt farblose Kristalle. Die Titelverbindung fiel als Gemisch von zwei Modifikationen mit Schmelzpunkten von 199 bis 200 °C bzw. 216 bis 217 °C an. Ausbeute: 79,2 %.

Allgemeine Vorschrift B zur Herstellung von 7-Aminoazolopyrimidinen der allgemeinen Formel IV

### Variante 1:

Eine Lösung von 97 mmol eines Ketonitrils gemäß Vorschrift A wird in 60 ml Mesitylen gelöst und zusammen mit 8,1 g (97 mmol) 3-Amino-1,2,4-triazol (bzw. 97 mmol 3-Aminopyrazol) und 3,8 g 4-Toluolsulfonsäure für drei Stunden auf 180 °C erhitzt, wobei etwas Lösungsmittel und das Reaktionswasser abdestilliert. Dann wird das Lösungsmittel vollständig abdestilliert und der Rückstand wird mit Methylenchlorid aufgenommen. Nach Waschen mit gesättigter, wässriger NaHCO₃-Lösung und Wasser wird die organische Phase getrocknet, vom Lösungsmittel befreit und der Rückstand wird mit Diethylether digeriert. Die Verbindungen der Formel IV verbleiben als farblose bis blassgelbe Kristalle oder als farblose bis blassgelbe Öle.

### Variante 2:

60 ml o-Xylol werden bei Raumtemperatur mit Oleum versetzt (18,4 mmol SO₃) und anschließend zusammen mit 97 mmol eines Ketonitrils gemäß Vorschrift A, 8,1 g (97 mmol) 3-Amino-1,2,4-triazol (bzw. 97 mmol 3-Aminopyrazol) und 3,8 g 4-Toluolsulfonsäure für acht Stunden auf Rückfluss erhitzt und dabei das Reaktionswasser abgetrennt. Dann wird das Lösungsmittel vollständig abdestilliert und der Rückstand mit Methylenchlorid aufgenommen. Nach Waschen mit gesättigter NaHCO₃-Lösung und Wasser wird die organische Phase getrocknet, vom Lösungsmittel befreit und der Rückstand mit Diethylether digeriert. Die Verbindungen der Formel IV verbleiben als farblose bis blassgelbe Kristalle oder als farblose bis blassgelbe Öle.

### Variante 3: 60 ml o-Xylol werden bei Raumtemperatur mit 2,14 g (18,4 mmol) Chlorsulfonsäure versetzt und anschließend zusammen mit 97 mmol eines Ketonitrils gemäß Vorschrift A, 8,1 g (97 mmol) 3-Amino-1,2,4-triazol (bzw. 97 mmol 3-Aminopyrazol) und 3,8 g 4-Toluolsulfonsäure für zehn Stunden auf Rückfluss erhitzt und das entstehende Reaktionswasser ausgekreist. Der Ansatz wird dann über einen Zeitraum von 10 h auf 20 °C abgekühlt. Die entstandene Suspension wird abfiltriert und der Filterkuchen abschließend mit Methanol und Wasser gewaschen. Nach dem Trocknen im Vakuum erhält man die Verbindungen der Formel IV als farblose bis blassgelbe Kristalle oder als farblose bis blassgelbe Öle.

Auf diese Weise können die folgenden Verbindungen der Formel IV hergestellt werden, soweit nichts anderes angegeben ist:

| Bsp. | R¹ | R² | x | R⁴ |
|---|---|---|---|---|
| 52 | CH₂CH₂CH₂CH₂CH₂CH₃ | CH₃ | N | H |
| 53 | CH₂CH(CH₃)CH₂CH₂CH₃ | CH₃ | N | H |
| 54 | CH₂CH₂CH₂CH₂CH₂CH₂CH₃ | CH₃ | N | H |
| 55 | CH₂CH₂CH(CH₃)CH₂C(CH₃)₃ | CH₃ | N | H |
| 56 | CH₂CH₂C(CH₃)₂CH₂C(CH₃)₃ | CH₃ | N | H |
| 57 | CH₂CH₂CH₂CH₂CH₂CH₃ | CF₃* | N | H |
| 58 | CH₂CH(CH₃)CH₂CH₂CH₃ | CF₃* | N | H |
| 59 | CH₂CH₂CH₂CH₂CH₂CH₂CH₃ | CF₃* | N | H |
| 60 | CH₂CH₂CH₂CH₂CH₂CH₂CH₂CH₃ | CF₃* | N | H |
| 61 | CH₂CH₂CH(CH₃)CH₂C(CH₃)₃ | CF₃* | N | H |
| 62 | CH₂CH₂C(CH₃)₂CH₂C(CH₃)₃ | CF₃* | N | H |
| 63 | CH₂CH₂CH₂CH₂CH₂CH₃ | CH₂CH₃ | N | H |
| 64 | CH₂CH(CH₃)CH₂CH₂CH₃ | CH₂CH₃ | N | H |
| 65 | CH₂CH₂CH₂CH₂CH₂CH₂CH₃ | CH₂CH₃ | N | H |
| 66 | CH₂CH₂CH(CH₃)CH₂C(CH₃)₃ | CH₂CH₃ | N | H |
| 67 | CH₂CH₂C(CH₃)₂CH₂C(CH₃)₃ | CH₂CH₃ | N | H |
| 68 | CH₂CH₂CH₂CH₂CH₂CH₃ | CH₂CH₂CH₃ | N | H |
| 69 | CH₂CH(CH₃)CH₂CH₂CH₃ | CH₂CH₂CH₃ | N | H |
| 70 | CH₂CH₂CH₂CH₂CH₂CH₂CH₃ | CH₂CH₂CH₃ | N | H |
| 71 | CH₂CH₂CH₂CH₂CH₂CH₂CH₂CH₃ | CH₂CH₂CH₃ | N | H |
| 72 | CH₂CH₂CH(CH₃)CH₂C(CH₃)₃ | CH₂CH₂CH₃ | N | H |
| 73 | CH₂CH₂C(CH₃)₂CH₂C(CH₃)₃ | CH₂CH₂CH₃ | N | H |
| 74 | CH₂CH₂CH₂CH₂CH₂CH₃ | CH₂OCH₃* | N | H |
| 75 | CH₂CH(CH₃)CH₂CH₂CH₃ | CH₂OCH₃* | N | H |
| 76 | CH₂CH₂CH₂CH₂CH₂CH₂CH₃ | CH₂OCH₃* | N | H |
| 77 | CH₂CH₂CH(CH₃)CH₂C(CH₃)₃ | CH₂OCH₃* | N | H |
| 78 | CH₂CH₂C(CH₃)₂CH₂C(CH₃)₃ | CH₂OCH₃* | N | H |
| 79 | CH₂CH₂CH₂CH₂CH₂CH₃ | CH₃ | N | NH₂ |
| 80 | CH₂CH(CH₃)CH₂CH₂CH₃ | CH₃ | N | NH₂ |
| 81 | CH₂CH₂CH₂CH₂CH₂CH₂CH₃ | CH₃ | N | NH₂ |
| 82 | CH₂CH₂CH₂CH₂CH₂CH₂CH₂CH₃ | CH₃ | N | NH₂ |
| 83 | CH₂CH₂CH(CH₃)CH₂C(CH₃)₃ | CH₃ | N | NH₂ |
| 84 | CH₂CH₂C(CH₃)₂CH₂C(CH₃)₃ | CH₃ | N | NH₂ |
| 85 | CH₂CH₂CH₂CH₂CH₂CH₃ | CF₃^{*} | N | NH₂ |
| 86 | CH₂CH(CH₃)CH₂CH₂CH₃ | CF₃^{*} | N | NH₂ |
| 87 | CH₂CH₂CH₂CH₂CH₂CH₂CH₃ | CF₃* | N | NH₂ |
| 88 | CH₂CH₂CH₂CH₂CH₂CH₂CH₂CH₃ | CF₃* | N | NH₂ |
| 89 | CH₂CH₂CH(CH₃)CH₂C(CH₃)₃ | CF₃* | N | NH₂ |
| 90 | CH₂CH₂C(CH₃)₂CH₂C(CH₃)₃ | CF₃* | N | NH₂ |
| 91 | CH₂CH₂CH₂CH₂CH₂CH₃ | CH₂CH₃ | N | NH₂ |
| 92 | CH₂CH(CH₃)CH₂CH₂CH₃ | CH₂CH₃ | N | NH₂ |
| 93 | CH₂CH₂CH₂CH₂CH₂CH₂CH₃ | CH₂CH₃ | N | NH₂ |
| 94 | CH₂CH₂CH₂CH₂CH₂CH₂CH₂CH₃ | CH₂CH₃ | N | NH₂ |
| 95 | CH₂CH₂CH(CH₃)CH₂C(CH₃)₃ | CH₂CH₃ | N | NH₂ |
| 96 | CH₂CH₂C(CH₃)₂CH₂C(CH₃)₃ | CH₂CH₃ | N | NH₂ |
| 97 | CH₂CH₂CH₂CH₂CH₂CH₃ | CH₂CH₂CH₃ | N | NH₂ |
| 98 | CH₂CH(CH₃)CH₂CH₂CH₃ | CH₂CH₂CH₃ | N | NH₂ |
| 99 | CH₂CH₂CH₂CH₂CH₂CH₂CH₃ | CH₂CH₂CH₃ | N | NH₂ |
| 100 | CH₂CH₂CH₂CH₂CH₂CH₂CH₂CH₃ | CH₂CH₂CH₃ | N | NH₂ |
| 101 | CH₂CH₂CH(CH₃)CH₂C(CH₃)₃ | CH₂CH₂CH₃ | N | NH₂ |
| 102 | CH₂CH₂C(CH₃)₂CH₂C(CH₃)₃ | CH₂CH₂CH₃ | N | NH₂ |
| 103 | CH₂CH₂CH₂CH₂CH₂CH₃ | CH₂OCH₃* | N | NH₂ |
| 104 | CH₂CH(CH₃)CH₂CH₂CH₃ | CH₂OCH₃* | N | NH₂ |
| 105 | CH₂CH₂CH₂CH₂CH₂CH₂CH₃ | CH₂OCH₃* | N | NH₂ |
| 106 | CH₂CH₂CH₂CH₂CH₂CH₂CH₂CH₃ | CH₂OCH₃* | N | NH₂ |
| 107 | CH₂CH₂CH(CH₃)CH₂C(CH₃)₃ | CH₂OCH₃* | N | NH₂ |
| 108 | CH₂CH₂C(CH₃)₂CH₂C(CH₃)₃ | CH₂OCH₃* | N | NH₂ |
| 109 | CH₂CH₂CH₂CH₂CH₂CH₃ | CH₃ | CH | H |
| 110 | CH₂CH(CH₃)CH₂CH₂CH₃ | CH₃ | CH | H |
| 111 | CH₂CH₂CH₂CH₂CH₂CH₂CH₃ | CH₃ | CH | H |
| 112 | CH₂CH₂CH₂CH₂CH₂CH₂CH₂CH₃ | CH₃ | CH | H |
| 113 | CH₂CH₂CH(CH₃)CH₂C(CH₃)₃ | CH₃ | CH | H |
| 114 | CH₂CH₂C(CH₃)₂CH₂C(CH₃)₃ | CH₃ | CH | H |
| 115 | CH₂CH₂CH₂CH₂CH₂CH₃ | CF₃* | CH | H |
| 116 | CH₂CH(CH₃)CH₂CH₂CH₃ | CF₃* | CH | H |
| 117 | CH₂CH₂CH₂CH₂CH₂CH₂CH₃ | CF₃* | CH | H |
| 118 | CH₂CH₂CH₂CH₂CH₂CH₂CH₂CH₃ | CF₃* | CH | H |
| 119 | CH₂CH₂CH(CH₃)CH₂C(CH₃)₃ | CF₃* | CH | H |
| 120 | CH₂CH₂C(CH₃)₂CH₂C(CH₃)₃ | CF₃* | CH | H |
| 121 | CH₂CH₂CH₂CH₂CH₂CH₃ | CH₂CH₃ | CH | H |
| 122 | CH₂CH(CH₃)CH₂CH₂CH₃ | CH₂CH₃ | CH | H |
| 123 | CH₂CH₂CH₂CH₂CH₂CH₂CH₃ | CH₂CH₃ | CH | H |
| 124 | CH₂CH₂CH₂CH₂CH₂CH₂CH₂CH₃ | CH₂CH₃ | CH | H |
| 125 | CH₂CH₂CH(CH₃)CH₂C(CH₃)₃ | CH₂CH₃ | CH | H |
| 126 | CH₂CH₂C(CH₃)₂CH₂C(CH₃)₃ | CH₂CH₃ | CH | H |
| 127 | CH₂CH₂CH₂CH₂CH₂CH₃ | CH₂CH₂CH₃ | CH | H |
| 128 | CH₂CH(CH₃)CH₂CH₂CH₃ | CH₂CH₂CH₃ | CH | H |
| 129 | CH₂CH₂CH₂CH₂CH₂CH₂CH₃ | CH₂CH₂CH₃ | CH | H |
| 130 | CH₂CH₂CH₂CH₂CH₂CH₂CH₂CH₃ | CH₂CH₂CH₃ | CH | H |
| 131 | CH₂CH₂CH(CH₃)CH₂C(CH₃)₃ | CH₂CH₂CH₃ | CH | H |
| 132 | CH₂CH₂C(CH₃)₂CH₂C(CH₃)₃ | CH₂CH₂CH₃ | CH | H |
| 133 | CH₂CH₂CH₂CH₂CH₂CH₃ | CH₂OCH₃* | CH | H |
| 134 | CH₂CH(CH₃)CH₂CH₂CH₃ | CH₂OCH₃* | CH | H |
| 135 | CH₂CH₂CH₂CH₂CH₂CH₂CH₃ | CH₂OCH₃* | CH | H |
| 136 | CH₂CH₂CH₂CH₂CH₂CH₂CH₂CH₃ | CH₂OCH₃* | CH | H |
| 137 | CH₂CH₂CH(CH₃)CH₂C(CH₃)₃ | CH₂OCH₃* | CH | H |
| 138 | CH₂CH₂C(CH₃)₂CH₂C(CH₃)₃ | CH₂OCH₃* | CH | H |
| 139 | CH₂CH=CH₂ | CH₃ | N | H |
| 140 | CH₂CH=CH₂ | CF₃* | N | H |
| 141 | CH₂CH=CH₂ | CH₂CH₂CH₃ | N | H |
| 142 | CH₂CH=CH₂ | CH₂OCH₃* | N | H |
| 143 | CH₂CH=CH₂ | (CH₂)₃CH₃ | N | H |
| 144 | CH₂CH=CH₂ | (CH₂)₄CH₃ | N | H |
| 145 | CH₂CH=CH₂ | (CH₂)₅CH₃ | N | H |
| 146 | CH₂CH=CH₂ | (CH₂)₆CH₃ | N | H |
| 147 | CH₂CH=CH₂ | (CH₂)₇CH₃ | N | H |
| 148 | CH₂CH=CH₂ | (CH₂)₈CH₃* | N | H |
| 149 | CH₂CH=CH₂ | CH₃ | CH | H |
| 150 | CH₂CH=CH₂ | CF₃* | CH | H |
| 151 | CH₂CH=CH₂ | CH₂CH₃ | CH | H |
| 152 | CH₂CH=CH₂ | CH₂CH₂CH₃ | CH | H |
| 153 | CH₂CH=CH₂ | CH₂OCH₃* | CH | H |
| 154 | CH₂CH=CH₂ | (CH₂)₃CH₃ | CH | H |
| 155 | CH₂CH=CH₂ | (CH₂)₄CH₃ | CH | H |
| 156 | CH₂CH=CH₂ | (CH₂)₅CH₃ | CH | H |
| 157 | CH₂CH=CH₂ | (CH₂)₆CH₃ | CH | H |
| 158 | CH₂CH=CH₂ | (CH₂)₇CH₃ | CH | H |
| 159 | CH₂CH=CH₂ | (CH₂)₈CH₃* | CH | H |

| | | | | |
|---|---|---|---|---|
| ²⁾ bezogen auf das Ketonitril der Formel I ^{*)} nicht erfindungsgemäßes Beispiel | | | | |

## Patentansprüche

1. Verfahren zur Herstellung von ß-Ketonitrilen der allgemeinen Formel I 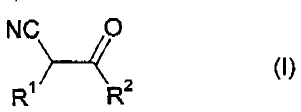 worin
R¹ für C₂-C₁₂-Alkyl, C₂-C₁₂-Alkenyl, C₂-C₁₂-Alkinyl, C₃-C₈-Cycloalkyl, C₃-C₈-Cycloalkyl-C₁-C₄-alkyl, Phenyl-C₁-C₄-alkyl, Phenoxy-C₁-C₄-alkyl oder Benzyloxy-C₁-C₄-alkyl steht, wobei C₂-C₁₂-Alkyl durch einen der Reste R^{A} substituiert sein kann, die C₃-C₈-Cycloalkylgruppen in den vorgenannten Resten durch 1, 2, 3, 4 oder 5 Reste R^{B} substituiert sein können und die Phenylgruppen in den vorgenannten Resten durch 1, 2, 3, 4 oder 5 Reste R^{C} substituiert sein können;
R^{A} ausgewählt ist unter C₁-C₁₀-Alkoxy, C₁-C₆-Alkoxy-C₁-C₄-alkoxy, C₁-C₁₀-Alkylthio, NH₂, C₁-C₁₀-Alkylamino und Di-C₁-C₁₀-alkylamino;
R^{B} ausgewählt ist unter Fluor, C₁-C₄-alkyl, C₁-C₄-Perfluoralkyl und den für R^{A} angegebenen Bedeutungen;
R^{C} ausgewählt ist unter Fluor, Nitro, C₁-C₄-alkyl, C₁-C₄-Perfluoralkyl und den für R^{A} angegebenen Bedeutungen,
wobei R¹ wenigstens 3 C-Atome aufweist;
R² für C₁-C₈-Alkyl steht;
umfassend die Umsetzung eines Nitrils der Formel II 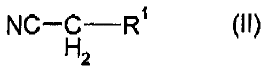 worin R¹ eine der zuvor genannten Bedeutungen aufweist, mit einem Carbonsäureester der Formel III, 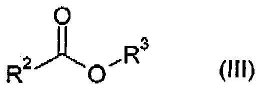 worin R² eine der zuvor genannten Bedeutungen aufweist und R³ für C₁-C₁₂-Alkyl, C₁-C₄-Alkoxy-C₁-C₄-alkyl, C₃-C₈-Cycloalkyl, C₃-C₈-Cyrloalkyl-C₁-C₄-alkyl, Phenyl-C₁-C₄-alkyl, Phenoxy-C₁-C₄-alkyl oder Benzyloxy-C₁-C₄-alkyl steht, wobei man die Umsetzung in einem inerten Lösungsmittel durchführt, das unter aliphatischen Kohlenwasserstoffen, cycloaliphatischen Kohlenwasserstoffen, aromatischen Kohlenwasserstoffen, Dialkylethern, alicyclischen Ethern und deren Gemischen aus gewählt ist,
wobei man die Umsetzung in Gegenwart eines Kaliumalkoholats durchführt und wenigstens 80 % des Nitrils der Formel II der Umsetzung unter Reaktionsbedingungen zuführt, wobei die Zugabe des Nitrils der Formel II und des Esters der Formel III parallel stattfindet.

2. Verfahren nach Anspruch 1, wobei man wenigstens 80 % des Carbonsäureesters der Formel III der Umsetzung unter Reaktionsbedingungen zuführt.

3. Verfahren nach einem der vorhergehenden Ansprüche, wobei man 1 bis 20 % des Esters der Formel III vorlegt.

4. Verfahren nach einem der vorhergehenden Ansprüche, wobei das inerte Lösungsmittel zu wenigstens 80 Gew.-%, bezogen auf die Gesamtmenge an inertem Lösungsmittel, aus einem oder mehreren Alkylaromaten oder aus einer Mischung von wenigstens einem Alkylaromaten mit einem oder mehreren aliphatischen oder cycloaliphatischen Lösungsmitteln besteht.

5. Verfahren nach einem der vorhergehenden Ansprüche, wobei man 1 bis 20 Mol-% wenigstens eines C₁-C₄-Alkanols, bezogen auf die Gesamtmenge des Esters der Formel III, vorlegt.

6. Verfahren nach einem der vorhergehenden Ansprüche, wobei das Molverhältnis von Kaliumalkoholat zu der Gesamtmenge an Nitril der Formel II wenigstens 0,9 : 1 beträgt.

7. Verfahren nach einem der vorhergehenden Ansprüche, wobei R¹ in den Formeln I und II für C₆-C₁₂-Alkyl steht.

8. Verfahren nach einem der vorhergehenden Ansprüche, wobei R³ in Formel III für C₁-C₈-Alkyl oder C₁-C₄-Alkoxy-C₁-C₄-alkyl steht.

9. Verfahren zur Herstellung von Azolopyrimidinen der Formel IV 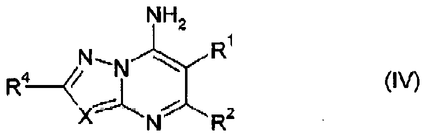 worin R¹ und R² eine der in Anspruch 1 angegebenen Bedeutungen aufweisen,
⁴ R für Wasserstoff, NH₂ oder C₁-C₆-Alkyl steht und
X für N oder eine Gruppe C-R⁵ steht, worin R⁵ für Wasserstoff oder C₁-C₆-Alkyl steht;
umfassend:
a) Herstellung eines ß-Ketonitrils der Formel I gemäß einem Verfahren nach einem der Ansprüche 1 bis 10 und
b) Umsetzung des ß-Ketonitrils der Formel I mit einer Aminoazolverbindung der Formel V 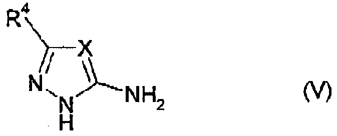 worin R⁴ und X die zuvor genannten Bedeutungen aufweisen, oder einem Tautomer davon in an sich bekannter Weise, wobei man das Azolopyrimidin der Formel IV erhält.

10. Verfahren nach Anspruch 9, wobei man das das in Stufe a) erhaltene Reaktionsgemisch, welches das ß-Ketonitril der Formel II enthält, neutralisiert und ohne weitere Aufreinigung in Stufe b) mit der Aminoazolverbindung der Formel V umsetzt.

## Claims

1. A process for preparing ß-ketonitriles of the general formula I 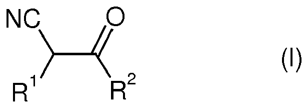 in which
R¹ is C₂-C₁₂-alkyl, C₂-C₁₂-alkenyl, C₂-C₁₂-alkinyl, C₃-C₈-cycloalkyl, C₃-C₈-cycloalkyl-C₁-C₄-alkyl, phenyl-C₁-C₄-alkyl, phenoxy-C₁-C₄-alkyl or benzyloxy-C₁-C₄-alkyl, where C₂-C₁₂-alkyl may be substituted by one of the radicals R^{A}, the C₃-C₈-cycloalkyl groups in the radicals mentioned above may be substituted by 1, 2, 3, 4 or 5 radicals R^{B} and the phenyl groups in the radicals mentioned above may be substituted by 1, 2, 3, 4 or 5 radicals R^{C};
R^{A} is selected from the group consisting of C₁-C₁₀-alkoxy, C₁-C₆-alkoxy-C₁-C₄-alkoxy, C₁-C₁₀-alkylthio, NH₂, C₁-C₁₀-alkylamino and di-C₁-C₁₀-alkylamino;
R^{B} is selected from the group consisting of fluorine, C₁-C₄-alkyl, C₁-C₄-perfluoroalkyl and the meanings given for R^{A};
R^{C} is selected from the group consisting of fluorine, nitro, C₁-C₄-alkyl, C₁-C₄-perfluoroalkyl and the meanings given for R^{A}, where R¹ has at least 3 carbon atoms;
R² is C₁-C₈-alkyl;
which comprises reacting a nitrile of the formula II 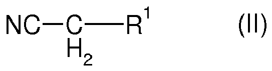 in which R¹ has one of the meanings given above with a carboxylic ester of the formula III 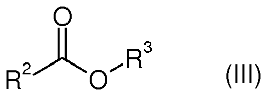 in which R² has one of the meanings given above and R³ is C₁-C₁₂-alkyl, C₁-C₄-alkoxy-C₁-C₄-alkyl, C₃-C₈-cycloalkyl, C₃-C₈-cycloalkyl-C₁-C₄-alkyl, phenyl-C₁-C₄-alkyl, phenoxy-C₁-C₄-alkyl or benzyloxy-C₁-C₄-alkyl,
where the reaction is carried out in an inert solvent which is selected from the group consisting of aliphatic hydrocarbons, cycloaliphatic hydrocarbons, aromatic hydrocarbons, dialkyl ethers, alicyclic ethers and mixtures thereof,
where the reaction is carried out in the presence of a potassium alkoxide and at least 80% of the nitrile of the formula II are added to the reaction under reaction conditions, where the addition of the nitrile of the formula II and of the ester of the formula III takes place in parallel.

2. The process according to claim 1 where at least 80% of the carboxylic ester of the formula III are added to the reaction under reaction conditions.

3. The process according to any of the preceding claims where 1 to 20% of the ester of the formula III are initially charged.

4. The process according to any of the preceding claims where the inert solvent comprises at least 80% by weight, based on the total amount of inert solvent, of one or more alkylaromatics or a mixture of at least one alkylaromatic with one or more aliphatic or cycloaliphatic solvents.

5. The process according to any of the preceding claims where 1 to 20 mmol% of at least one C₁-C₄-alkanol, based on the total amount of the ester of the formula III, is initially charged.

6. The process according to any of the preceding claims where the molar ratio of potassium alkoxide to the total amount of nitrile of the formula II is at least 0.9 : 1.

7. The process according to any of the preceding claims where R¹ in the formulae I and II is C₆-C₁₂-alkyl.

8. The process according to any of the preceding claims where R³ in formula III is C₁-C₈-alkyl or C₁-C₄-alkoxy-C₁-C₄-alkyl.

9. A process for preparing azolopyrimidines of the formula IV 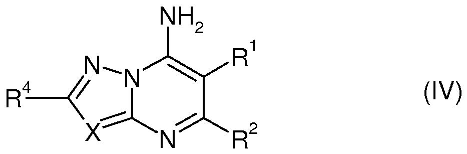 in which R¹ and R² have one of the meanings given in claim 1,
R⁴ is hydrogen, NH₂ or C₁-C₆-alkyl and
X is N or a group C-R⁵, where R⁵ is hydrogen or C₁-C₆-alkyl ;
which comprises:
a) preparing a ß-ketonitrile of the formula I by a process according to any one of claims 1 to 10 and
b) reacting the ß-ketonitrile of the formula I with an aminoazole compound of the formula V 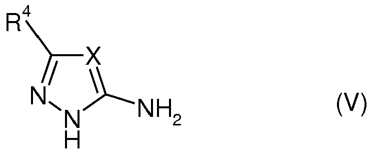 in which R⁴ and X have the meanings mentioned above, or a tautomer thereof, in a known manner, giving the azolopyrimidine of the formula IV.

10. The process according to claim 9 where the reaction mixture obtained in step a), which comprises the ß-ketonitrile of the formula II, is neutralized and, without further purification, reacted in step b) with the aminoazole compound of the formula V.

## Revendications

1. Procédé pour la préparation de ß-cétonitriles de formule générale I 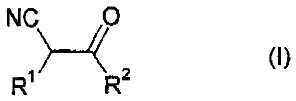 où
R¹ représente C₂-C₁₂-alkyle, C₂-C₁₂-alcényle, C₂-C₁₂-alcynyle, C₃-C₈-cycloalkyle, C₃-C₈-cycloalkyl-C₁-C₄-alkyle, phényl-C₁-C₄-alkyle, phénoxy-C₁-C₄-alkyle ou benzyloxy-C₁-C₄-alkyle, où C₂-C₁₂-alkyle peut être substitué par un des radicaux R^{A}, les groupes C₃-C₈-cycloalkyle dans les radicaux susmentionnés peuvent être substitués par 1, 2, 3, 4 ou 5 radicaux R^{B} et les groupe phényle dans les radicaux susmentionnés peuvent être substitués par 1, 2, 3, 4 ou 5 radicaux R^{C} ;
R^{A} est choisi parmi C₁-C₁₀-alcoxy, C₁-C₆-alcoxy-C₁-C₄-alcoxy, C₁-C₁₀-alkylthio, NH₂, C₁-C₁₀-alkylamino et di-C₁-C₁₀-alkylamino ;
R^{B} est choisi parmi fluor, C₁-C₄-alkyle, C₁-C₄-perfluoroalkyle et les significations indiquées pour R^{A} ;
R^{C} est choisi parmi fluor, nitro, C₁-C₄-alkyle, C₁-C₄-perfluoroalkyle et les significations indiquées pour R^{A},
R¹ présentant au moins 3 atomes de carbone ;
R² représente C₁-C₈-alkyle ;
comprenant la transformation d'un nitrile de formule II 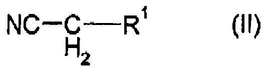 dans laquelle R¹ présente une des significations susmentionnées, avec un ester d'acide carboxylique de formule III, 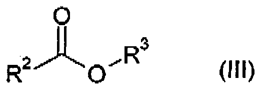 dans laquelle R² présente une des significations susmentionnées et R³ représente C₁-C₁₂-alkyle, C₁-C₄-alcoxy-C₁-C₄-alkyle, C₃-C₈-cycloalkyle, C₃-C₈-cycloalkyl-C₁-C₄-alkyle, phényl-C₁-C₄-alkyle, phénoxy-C₁-C₄-alkyle ou benzyloxy-C₁-C₄-alkyle,
la transformation étant réalisée dans un solvant inerte, qui est choisi parmi les hydrocarbures aliphatiques, les hydrocarbures cycloaliphatiques, les hydrocarbures aromatiques, les dialkyléthers, les éthers alicycliques et leurs mélanges,
la transformation étant réalisée en présence d'un alcoolate de potassium et en alimentant au moins 80% du nitrile de formule II dans la transformation dans des conditions de réaction, l'addition du nitrile de formule II et de l'ester de formule III ayant lieu en parallèle.

2. Procédé selon la revendication 1, au moins 80% de l'ester de l'acide carboxylique de formule III étant alimenté dans la transformation dans des conditions de réaction.

3. Procédé selon l'une quelconque des revendications précédentes, 1 à 20% de l'ester de formule III étant disposés au préalable.

4. Procédé selon l'une quelconque des revendications précédentes, le solvant inerte étant constitué à raison d'au moins 80% en poids, par rapport à la quantité totale de solvant inerte, d'un ou de plusieurs aromatiques d'alkyle ou d'un mélange d'au moins un aromatique d'alkyle et d'un ou de plusieurs solvants aliphatiques ou cycloaliphatiques.

5. Procédé selon l'une quelconque des revendications précédentes, 1 à 20% en mole d'au moins un C₁-C₄-alcanol, par rapport à la quantité totale de l'ester de formule III, étant disposés au préalable.

6. Procédé selon l'une quelconque des revendications précédentes, le rapport molaire d'alcoolate de potassium à la quantité totale de nitrile de formule II valant au moins 0,9:1.

7. Procédé selon l'une quelconque des revendications précédentes, R¹ dans les formules I et II représentant C₆-C₁₂-alkyle.

8. Procédé selon l'une quelconque des revendications précédentes, R³ dans la formule III représentant C₁-C₈-alkyle ou C₁-C₄-alcoxy-C₁-C₄-alkyle.

9. Procédé pour la préparation d'azolopyrimidines de formule IV 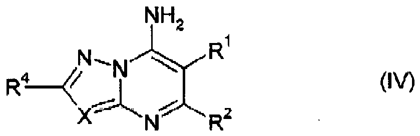 dans laquelle R¹ et R² présentent une des significations indiquées dans la revendication 1,
R⁴ représente hydrogène, NH₂ ou C₁-C₈-alkyle et
X représente N ou un groupe C-R⁵, dans lequel R⁵ représente hydrogène ou C₁-C₆-alkyle ;
comprenant
a) la préparation d'un ß-cétonitrile de formule I selon un procédé selon l'une quelconque des revendications 1 à 10 et
b) transformation du ß-cétonitrile de formule I avec un composé aminoazole de formule V, 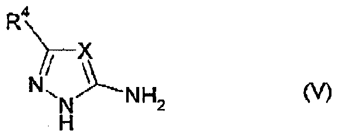 dans laquelle R⁴ et X présentent les significations susmentionnées, ou un tautomère de celui-ci, de manière connue en soi, en obtenant l'azolopyrimidine de formule IV.

10. Procédé selon la revendication 9, le mélange réactionnel obtenu dans l'étape a), qui contient le ß-cétonitrile de formule II, étant neutralisé et transformé sans autre purification dans l'étape b) avec le composé aminoazole de formule V.
